# EUROPEAN PATENT APPLICATION

(11) **EP 4 163 294 A1**
(43) Date of publication of application: **12.04.2023**
(21) Application number: 21822419.4
(22) Date of filing: 08.06.2021
(51) Int. Cl.: C07K 14/47, C12N 15/12

(54) **MODIFIED FERRITIN AND METHOD FOR PRODUCING SAME**

(30) Priority: 09.06.2020 JP 2020100456
(71) Applicant: AJINOMOTO CO., INC., Chuo-ku Tokyo 104-8315 (JP)
(72) Inventor: YAMADA, Kei, Kawasaki-shi, Kanagawa 210-8681 (JP); INOUE, Ippei, Kawasaki-shi, Kanagawa 210-8681 (JP); SHIKIDA, Natsuki, Kawasaki-shi, Kanagawa 210-8681 (JP); SHIMBO, Kazutaka, Kawasaki-shi, Kanagawa 210-8681 (JP)
(74) Representative: Mewburn Ellis LLP
(86) International application number: PCT/JP2021/021673
(87) International publication number: WO 2021/251358

(57) **Abstract**

The present invention provides a technique which can easily control the number of modifying groups introduced into a surface of ferritin. More specifically, the present invention provides a modified ferritin containing a human ferritin H chain, in which the human ferritin H chain contains a modifying group that is covalently bonded specifically to a cysteine residue at position 91 and/or position 103 according to a reference position of a natural human ferritin H chain; and the like.

## Description

### Field

The present invention relates to a modified ferritin and a method for producing the same.

### Background

Ferritin is a spherical protein that is ubiquitously present in organisms from animals and plants to microorganism and has an internal cavity constituted by a plurality of monomers. In animals such as humans, it is known that two types of monomers of H and L chains are present as monomers constituting ferritin, and that ferritin is a multimer that is constituted by 24 monomers (in many cases, a mixture of H and L chains) and has a cage-like form having an outer diameter of 12 nm and an internal cavity having an inner diameter of 7 nm. In addition, an N-terminal of a monomer constituting ferritin is exposed on a surface of a 24-mer, but a C-terminal of the monomer is not exposed on the surface and is present in the internal cavity. Ferritin can hold iron in its internal cavity, plays a role of a physiological function such as transport and storage of iron, and is thereby deeply involved in homeostasis of an iron element in a living body or a cell. Ferritin is relatively easily capable of dissociation and association, and is therefore in a form of a 24-mer or a monomer, or in a form in which the 24-mer and the monomer coexist depending on conditions of a ferritin-containing solvent and the like. Therefore, by appropriately adjusting conditions of ferritin-containing solvent and the like, the 24-mer can be dissociated into monomers, and the dissociated monomers can be reassociated to the 24-mer. In addition, it is also known that by causing a certain substance to coexist with the ferritin monomer in a solvent at the time of reassociation, the substance can be encapsulated in the internal cavity of the reassociated 24-mer.

Currently, a technique for modifying a surface of ferritin for the purpose of, for example, modifying deliverability of ferritin and improving stability of ferritin is known. For example, a technique using a modifying group capable of specifically reacting with an amino group (-NH₃) in a side chain of a lysine residue exposed on a surface of ferritin (Non Patent Literatures 1 and 2), and a technique using a modifying group capable of specifically reacting with an aspartic acid residue and a carboxyl group (-COOH) in a side chain of a glutamic acid residue exposed on a surface of ferritin (Patent Literature 1) have been reported.

In addition, a technique for modifying a surface of ferritin using a ferritin monomer into which a mutation enabling modification of the surface of ferritin is introduced is known. For example, as such a technique, a technique using a peptide added to an N-terminal of a ferritin monomer (Non Patent Literature 3) and a technique using a thiol group (-SH) in a cysteine residue introduced into a ferritin monomer (Non Patent Literature 4) have been reported.

### Citation List

### Patent Literature

Patent Literature 1: US 2008/0292545 A

### Non Patent Literature

Non Patent Literature 1: Xin Lin et al., Nano Letters(2011), 11(2), 814-819
Non Patent Literature 2: JOHN R. FEAGLER et al., J. Cell Biology(1974), 60(3), 541-53
Non Patent Literature 3: Dong Men et al., ACS Nano(2015), 9(11), 10852-10860
Non Patent Literature 4: Tuo Zhang et al., Adv. Mater. Research(2014), 887-888, 596-600

### Summary

### Technical Problem

The present inventors have focused on a fact that it may be difficult to control the number of modifying groups to be introduced into a surface of ferritin in a technique using a modifying group capable of specifically reacting with an amino group (-NH₃) in a side chain of a lysine residue exposed on a surface of ferritin, or a carboxyl group (-COOH) in a side chain of an aspartic acid residue and a glutamic acid residue exposed on a surface of ferritin (Non Patent Literatures 1 and 2 and Patent Literature 1). This is because a large number of lysine residues, aspartic acid residues, and glutamic acid residues are present in ferritin, these amino acid residues are exposed on a surface of ferritin, a large number of modifying groups can be introduced into the surface of ferritin, and therefore it is not easy to control the number of modifying groups. Since clinical application of ferritin requires high quality control, a modified ferritin excellent in uniformity of surface modification is desirable as compared with a modified ferritin that is not uniform. Therefore, development of a technique capable of reproducibly producing a modified ferritin excellent in uniformity of surface modification is desired. However, when the number of modifying groups cannot be easily controlled, it is not easy to reproducibly produce a modified ferritin excellent in uniformity of surface modification.

Therefore, an object of the present invention is to provide a technique capable of easily controlling the number of modifying groups introduced into a surface of ferritin.

### Solution to Problem

As a result of intensive studies, the present inventors have found that by using a thiol group (-SH) in a side chain of a cysteine residue of a human ferritin H chain which is a ferritin monomer as a reaction target, it is possible to selectively modify a thiol residue in a side chain of a cysteine residue at position 91 and/or position 103 according to a reference position of a natural human ferritin H chain, and consequently to easily and highly control the number of modifying groups introduced into a surface of ferritin. Non Patent Literatures 1 and 2 and Patent Literature 1 neither teach nor suggest the technical idea of controlling the number of modifying groups introduced into a surface of ferritin. Therefore, the present inventors have succeeded in reproducibly providing a modified ferritin excellent in uniformity of surface modification based on such findings and the like, and have completed the present invention.

That is, the present invention is as follows.
[1] A modified ferritin containing
   a human ferritin H chain, in which
   the human ferritin H chain contains a modifying group covalently bonded specifically to a cysteine residue at position 91 and/or position 103 according to a reference position of a natural human ferritin H chain.
[2] The modified ferritin according to [1], containing one or two modifying groups per human ferritin H chain.
[3] The modified ferritin according to [1] or [2], which is a 24-mer containing 24 human ferritin H chains.
[4] The modified ferritin according to [3], containing 24 or 48 modifying groups.
[5] The modified ferritin according to any one of [1] to [4], in which the human ferritin H chain is as follows:
   (A) a protein comprising the amino acid sequence of SEQ ID NO: 1 or SEQ ID NO: 3;
   (B) a protein comprising an amino acid sequence comprising one or several mutations of amino acid residues selected from the group consisting of replacement, deletion, insertion, and addition of amino acid residues in the amino acid sequence of SEQ ID NO: 1 or SEQ ID NO: 3 and having a 24-mer forming ability; or
   (C) a protein comprising an amino acid sequence having 90% or more identity to the amino acid sequence of SEQ ID NO: 1 or SEQ ID NO: 3 and having a 24-mer forming ability.
[6] The modified ferritin according to any one of [1] to [5], in which the human ferritin H chain is a natural human ferritin H chain optionally having a deletion of a methionine residue at position 1.
[7] The modified ferritin according to any one of [1] to [6], in which the human ferritin H chain has a functional peptide inserted into a flexible linker region consisting of amino acid residues at positions 78 to 96 according to a reference position of a natural human ferritin H chain.
[8] The modified ferritin according to [7], in which the functional peptide is a peptide having a binding ability to a target material.
[9] The modified ferritin according to any one of [1] to [8], in which the modifying group contains a reactive group, and the modified ferritin is a reactive group-added ferritin.
[10] The modified ferritin according to [9], in which the reactive group is a bioorthogonal functional group for a protein.
[11] The modified ferritin according to [10], in which the bioorthogonal functional group contains one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety.
[12] The modified ferritin according to any one of [1] to [8], in which the modifying group contains a functional substance, and the modified ferritin is a functional substance-added ferritin.
[13] The modified ferritin according to [12], in which the functional substance contains one or more moieties selected from the group consisting of a peptide, a protein, a nucleic acid, a low molecular organic compound, a chelator, a sugar chain, a lipid, a high molecular polymer, and a metal.
[14] The modified ferritin according to any one of [1] to [13], containing a substance in an internal cavity thereof.
[15] A method for producing a reactive group-added ferritin, including
   reacting a human ferritin with a thiol modifying reagent to form a reactive group-added ferritin, in which
   the human ferritin and the reactive group-added ferritin each contain a human ferritin H chain, and
   the human ferritin H chain contained in the reactive group-added ferritin contains a reactive group-containing modifying group covalently bonded specifically to a cysteine residue at position 91 and/or position 103 according to a reference position of a natural human ferritin H chain.
[16] The method according to [15], in which the thiol modifying reagent contains one or more partial structures selected from the group consisting of a maleimide moiety, a benzyl halide moiety, an α-haloamide moiety, an α-haloketone moiety, an alkene moiety, an alkyne moiety, a fluoroaryl moiety, a nitroaryl moiety, a methylsulfonyloxadiazole moiety, and a disulfide moiety.
[17] A method for producing a functional substance-added ferritin, including
   reacting a human ferritin with a functional substance to form a functional substance-added ferritin, in which
   the human ferritin and the functional substance-added ferritin each contain a human ferritin H chain, and
   the human ferritin H chain contained in the functional substance-added ferritin contains a functional substance-containing modifying group covalently bonded specifically to a cysteine residue at position 91 and/or position 103 according to a reference position of a natural human ferritin H chain.
[18] A method for producing a functional substance-added ferritin, including:
   (1) reacting a human ferritin with a thiol modifying reagent to form a reactive group-added ferritin; and
   (2) reacting a reactive group-added ferritin with a functional substance to form a functional substance-added ferritin, in which
      the human ferritin, the reactive group-added ferritin, and the functional substance-added ferritin each contain a human ferritin H chain,
      the human ferritin H chain contained in the reactive group-added ferritin contains a reactive group-containing modifying group covalently bonded specifically to a cysteine residue at position 91 and/or position 103 according to a reference position of a natural human ferritin H chain, and
      the human ferritin H chain contained in the functional substance-added ferritin contains a functional substance-containing modifying group covalently bonded specifically to a cysteine residue at position 91 and/or position 103 according to the reference position of the natural human ferritin H chain.

### Effects of Invention

The present invention can reproducibly provide a modified ferritin excellent in uniformity of surface modification. In addition, by using a natural human ferritin as ferritin, immunogenicity can be avoided in clinical application to humans.

### Brief Description of Drawings

FIG. 1 is a diagram illustrating (A) an amino acid sequence of a natural human ferritin H chain (with methionine at position 1) (SEQ ID NO: 1), (B) a nucleotide sequence of a polynucleotide encoding a natural human ferritin H chain (with methionine at position 1) (SEQ ID NO: 2), and (C) an amino acid sequence of a natural human ferritin H chain (without methionine at position 1) (SEQ ID NO: 3).
FIG. 2 is a diagram illustrating ESI-TOFMS analysis of a natural human ferritin H chain specifically modified with ethyl maleimide (six equivalents).
FIG. 3 is a diagram illustrating analysis (MS spectrum) of a modified site of a natural human ferritin H chain specifically modified with ethyl maleimide.
FIG. 4 is a diagram illustrating analysis (CID spectrum) of a modified site of a natural human ferritin H chain specifically modified with ethyl maleimide.
FIG. 5 is a diagram illustrating ESI-TOFMS analysis of a natural human ferritin H chain specifically modified with p-azidophenacyl bromide (six equivalents).
FIG. 6 is a diagram illustrating ESI-TOFMS analysis of a natural human ferritin H chain specifically modified with p-azidophenacyl bromide (12 equivalents).
FIG. 7 is a diagram illustrating analysis (MS spectrum) of a modified site of a natural human ferritin H chain specifically modified with p-azidophenacyl bromide.
FIG. 8 is a diagram illustrating analysis (CID spectrum) of a modified site of a natural human ferritin H chain specifically modified with p-azidophenacyl bromide.
FIG. 9 is a diagram illustrating a comparison among peak area values at positions 91, 103, and 131 of a human ferritin H chain based on an analysis result of a modified site of a human ferritin H chain by LC-MS/MS.
FIG. 10 is a diagram illustrating ESI-TOFMS analysis of a natural human ferritin H chain specifically modified with p-azidophenacyl bromide (six equivalents).
FIG. 11 is a diagram illustrating ESI-TOFMS analysis of a natural human ferritin H chain specifically modified with DBCO-PEG4-maleimide (six equivalents).
FIG. 12 is a diagram illustrating ESI-TOFMS analysis of natural human ferritin H chains specifically modified with 1,3-dichloroacetone (six equivalents) and aminooxy-PEG5-azide (ten equivalents).
FIG. 13 is a diagram illustrating an MS spectrum (measured value: m/z 853.37134, theoretical value: 853.37194, trivalent) of a peptide fragment of a peptide KPDCDDWESGLNAMECALHLEK (SEQ ID NO: 9) consisting of 22 amino acid residues containing one modified site (1,3-dichloroacetone modification (+ 54.011 Da)) to a cysteine residue by trypsin digestion of a human ferritin H chain (Example 9).
FIG. 14 is a diagram illustrating a CID spectrum of a product ion of m/z 1206.78 (theoretical value: 1206.50) corresponding to divalent b21 indicating 1,3-dichloroacetone modification (+54.011 Da) of cysteine residues at amino acid positions 90 and 102 of a human ferritin H chain (Example 9).
FIG. 15 is a diagram illustrating a result of calculating a peak area of an extraction chromatogram of a peptide containing 1,3-dichloroacetone modification (+54.011 Da) to a cysteine residue in a trypsin digest of a human ferritin H chain using Xcalibur Qual Browser (Example 9). The horizontal axis represents a cysteine residue contained in a human ferritin H chain, and the vertical axis represents a peak area.
FIG. 16 is a diagram illustrating amide group modification by ESI-TOFMS.
FIG. 17 is a diagram illustrating an MS spectrum (measured value: m/z 873.38158, theoretical value: 873.38273, trivalent) of a peptide fragment of a peptide KPDCDDWESGLNAMECALHLEK (SEQ ID NO: 9) consisting of 22 amino acid residues containing one modified site (α-iodoacetamide modification (+57.021 Da)) to a cysteine residue by trypsin digestion of a human ferritin H chain (Example 11).
FIG. 18 is a diagram illustrating a CID spectrum of a product ion of m/z 1236.85 (theoretical value: 1236.52) corresponding to divalent b21 indicating α-iodoacetamide modification (+57.021 Da) of cysteine residues at amino acid positions 90 and 102 of a human ferritin H chain (Example 11).
FIG. 19 is a diagram illustrating a result of calculating a peak area of an extraction chromatogram of a peptide containing α-iodoacetamide modification (+57.021 Da) to a cysteine residue in a trypsin digest of a human ferritin H chain using Xcalibur Qual Browser (Example 11). The horizontal axis represents a cysteine residue contained in a human ferritin H chain, and the vertical axis represents a peak area.
FIG. 20 is a diagram illustrating dispersibility of an ethyl maleimide-modified ferritin by DLS.
FIG. 21 is a diagram illustrating dispersibility of an ethyl maleimide-modified ferritin by size exclusion column chromatography.
FIG. 22 is a diagram illustrating stability of a doxorubicin-encapsulating ethyl maleimide-modified ferritin by size exclusion column chromatography.
FIG. 23 is a diagram illustrating encapsulation of a fluorescently modified peptide in an ethyl maleimide-modified ferritin by size exclusion column chromatography.
FIG. 24 is a diagram illustrating modification of a fluorescently modified peptide-encapsulating ferritin with ethyl maleimide by ESI-TOFMS.
FIG. 25 is a diagram illustrating that a peptide encapsulated in ferritin is maintained even when ethyl maleimide modification is performed on a fluorescently modified peptide-encapsulating ferritin by size exclusion column chromatography.
FIG. 26 is a diagram illustrating modification of a double-stranded DNA-encapsulating ferritin with ethyl maleimide by ESI-TOFMS.
FIG. 27 is a diagram illustrating that a double-stranded DNA encapsulated in ferritin is maintained even when ethyl maleimide modification is performed on a double-stranded DNA-encapsulating ferritin by size exclusion column chromatography.
FIG. 28 is a diagram illustrating modification with a peptide having PEG-6 as a linker (YGRKKRRQRRR, TAT, SEQ ID NO: 13) or (RRRRRRRRR, R9, SEQ ID NO: 14) by ESI-TOFMS.
FIG. 29 is a diagram illustrating dispersibility of azidated FTH by DLS.
FIG. 30 is a diagram illustrating dispersibility of azidated FTH by size exclusion column chromatography.
FIG. 31 is a diagram illustrating that a fluorescently modified peptide is encapsulated in azidated FTH by size exclusion column chromatography.
FIG. 32 is a diagram illustrating electrophoretic images of samples obtained by modifying surfaces of natural FTH and azidated FTH with siRNA by SDS-PAGE.
FIG. 33 is a diagram illustrating dispersibility of a nucleic acid-modified ferritin by DLS.
FIG. 34 is a diagram illustrating dispersibility of a nucleic acid-modified ferritin by size exclusion column chromatography.
FIG. 35 is a diagram illustrating modification of a peptide-inserted human ferritin H chain with p-azidophenacyl bromide by ESI-TOFMS.
FIG. 36 is a diagram illustrating modification of a surface of natural FTH with N-succinimidyl 3-(acetylthio) propionate by ESI-TOFMS.

### Embodiments for carrying out the invention

The present invention provides a modified ferritin containing a human ferritin H chain, in which
the human ferritin H chain contains a modifying group covalently bonded specifically to a cysteine residue at position 91 and/or position 103.

The position of an amino acid residue in the human ferritin H chain (for example, a methionine residue at position 1, a cysteine residue at position 91, and a cysteine residue at position 103) is determined according to a reference position of a natural human ferritin H chain. The natural human ferritin H chain is typically a polypeptide comprising the amino acid sequence of SEQ ID NO: 1. In the present invention, the natural human ferritin H chain includes not only a polypeptide comprising the amino acid sequence of SEQ ID NO: 1 but also a naturally occurring variant thereof. The natural human ferritin H chain is preferably a polypeptide comprising the amino acid sequence of SEQ ID NO: 1.

The human ferritin H chain contained in the modified ferritin of the present invention may be a natural human ferritin H chain optionally having a deletion of a methionine residue at position 1. A natural human ferritin H chain not having a deletion of a methionine residue at position 1 corresponds to a polypeptide comprising the amino acid sequence of SEQ ID NO: 1. A natural human ferritin H chain having a deletion of a methionine residue at position 1 corresponds to a polypeptide comprising the amino acid sequence of SEQ ID NO: 3.

In a certain embodiment, the human ferritin H chain may be as follows:
(A) a protein comprising the amino acid sequence of SEQ ID NO: 1 or SEQ ID NO: 3;
(B) a protein comprising an amino acid sequence comprising one or several mutations of amino acid residues selected from the group consisting of replacement, deletion, insertion, and addition of amino acid residues in the amino acid sequence of SEQ ID NO: 1 or SEQ ID NO: 3 and having a 24-mer forming ability; or
(C) a protein comprising an amino acid sequence having 90% or more identity to the amino acid sequence of SEQ ID NO: 1 or SEQ ID NO: 3 and having a 24-mer forming ability.

In the protein (B), one or several amino acid residues can be modified by one, two, three, or four mutations selected from the group consisting of deletion, replacement, addition, and insertion of amino acid residues. The mutations of the amino acid residues may be introduced into one region or a plurality of different regions in the amino acid sequence. In the present invention, as replacement of an amino acid residue, replacement of an amino acid residue other than a cysteine residue with a cysteine residue cannot be intended. Also as addition and insertion of an amino acid residue, addition and insertion of a cysteine residue or a cysteine residue-containing region cannot be intended. The term "1 or several" refers to a number that does not impair the 24-mer forming ability. The number indicated by the term "one or several" is, for example, 1 to 20, preferably 1 to 15, more preferably 1 to 10, and still more preferably 1 to 5 (for example, 1, 2, 3, 4, or 5).

In the protein (C), the degree of identity to an amino acid sequence of interest is preferably 92% or more, more preferably 95% or more, still more preferably 97% or more, and most preferably 98% or more or 99% or more. The % identity of the amino acid sequence can be calculated using GENETYX Ver 13.1.1 software manufactured by GENETYX Corporation, and using a numerical value obtained by performing muscle alignment, ClustalW alignment, or Multiple sequence alignment using the entire length of a polypeptide portion encoded by ORF, and then performing calculation with setting of Gaps are taken into account.

The position of an amino acid residue into which a mutation is to be introduced in an amino acid sequence is apparent to a person skilled in the art, but may be identified with further reference to sequence alignment. Specifically, a person skilled in the art can 1) compare a plurality of amino acid sequences with each other, 2) clarify a relatively preserved region and a relatively non-preserved region, and then 3) predict a region capable of playing an important role for a function and a region incapable of playing an important role for a function from the relatively preserved region and the relatively non-preserved region, respectively, and can thus recognize a structure-function correlation. Therefore, a person skilled in the art can identify a position into which a mutation is to be introduced in an amino acid sequence by using sequence alignment, and can also identify a position of an amino acid residue into which a mutation is to be introduced in an amino acid sequence by using known secondary and tertiary structure information in combination. It is also preferable to introduce mutations into amino acid residues (amino acid residues that are not exposed to a surface and therefore are at low risk of developing immunogenicity) in a C-terminal region located inside a cage-like structure of ferritin and in another region under a normal environment from a viewpoint of avoiding a risk of developing immunogenicity. Into a position not exposed on a surface of ferritin, a mutation (for example, replacement, insertion, and addition to an N-terminal region) of a cysteine residue or a cysteine residue-containing peptide may be introduced, but preferably, the mutation (for example, replacement, insertion, and addition to an N-terminal region) of the cysteine residue or the cysteine residue-containing peptide is not introduced.

When an amino acid residue is mutated by replacement, the replacement of the amino acid residue may be preservative replacement. The term "preservative replacement" when used in the present specification refers to replacing a certain amino acid residue with an amino acid residue having a similar side chain. Families of the amino acid residue having a similar side chain are known in the field concerned. Examples of such families include an amino acid having a basic side chain (for example, lysine, arginine, or histidine), an amino acid having an acidic side chain (for example, aspartic acid or glutamic acid), an amino acid having an uncharged polar side chain (for example, glycine, asparagine, glutamine, serine, threonine, tyrosine, or cysteine), an amino acid having a nonpolar side chain (for example, alanine, valine, leucine, isoleucine, proline, phenylalanine, methionine, or tryptophan), an amino acid having a β-position-branched side chain (for example, threonine, valine, or isoleucine), an amino acid having an aromatic side chain (for example, tyrosine, phenylalanine, tryptophan, or histidine), an amino acid having a hydroxy group (for example, alcoholic or phenolic)-containing side chain (for example, serine, threonine, or tyrosine), and an amino acid having a sulfur-containing side chain (for example, cysteine or methionine). The preservative replacement of an amino acid may be preferably replacement between aspartic acid and glutamic acid, replacement among arginine, lysine, and histidine, replacement between tryptophan and phenylalanine, replacement between phenylalanine and valine, replacement among leucine, isoleucine, and alanine, or replacement between glycine and alanine.

In a certain embodiment, the human ferritin H chain in the proteins of (B) and (C) may be a human ferritin H chain further containing a functional peptide not containing a cysteine residue. In the present invention, genetic modification other than genetic modification for introducing a modifying group into a surface of ferritin is allowed, and therefore genetic modification for further containing a functional peptide is allowed. An N-terminal of a ferritin monomer of a higher organism is exposed on a surface of a 24-mer. Into an N-terminal of the human ferritin H chain which is a ferritin monomer of a higher organism, various functional peptides can be inserted while the 24-mer forming ability is maintained. The ferritin monomer of a higher organism has six α-helices highly conserved in various higher organisms. In the human ferritin H chain which is a ferritin monomer of a higher organism, as the six α-helices, there are 1) a first region consisting of amino acid residues at positions 15 to 42 (also referred to as region A), 2) a second region consisting of amino acid residues at positions 50 to 77 (also referred to as region B), 3) a third region consisting of amino acid residues at positions 97 to 124 (also referred to as region C), 4) a fourth region consisting of amino acid residues at positions 128 to 137 (also referred to as region D), 5) a fifth region consisting of amino acid residues at positions 139 to 159 (also referred to as region D), and 6) a sixth region consisting of amino acid residues at positions 165 to 174 (also referred to as region E). A region between these α-helices is called a flexible linker region, and it has been reported that various functional peptides can be inserted between the flexible linker regions while the 24-mer forming ability is maintained (for example, WO 2019/163871 A; US 2016/0060307 A; Jae Og Jeon et al., ACS Nano (2013), 7 (9), 7462-7471; Sooji Kim et al., Biomacromolecules (2016), 17 (3), 1150-1159; and Young Ji Kang et al., Biomacromolecules (2012), 13 (12), 4057-4064). For example, α-helices of the regions B and C in a ferritin monomer are well known in the field concerned, and a person skilled in the art can appropriately identify the positions of the α-helices of the regions B and C in ferritin monomers derived from various organisms. Therefore, a flexible linker region between the α-helices of the regions B and C into which a functional peptide is to be inserted in the present invention is also well known in the field concerned, and can be appropriately identified by a person skilled in the art. For example, as such an insertion position of a functional peptide into the human ferritin H chain, positions 78 to 96 (preferably positions 83 to 91) which are flexible linker regions between the second and third regions can be suitably used (for example, WO 2019/163871 A).

As the functional peptide, a peptide capable of adding any function to a target protein when being fused with the target protein can be used. Examples of such a peptide include a peptide having a binding ability to a target material, a protease-degradable peptide, a cell penetrating peptide, and a stabilized peptide.

The functional peptide inserted into the region described above may be formed of only one peptide having a desired function, or may be formed of a plurality of peptides (several peptides such as 2, 3 or 4 peptides) of the same or different types having a desired function. When the functional peptide is formed of a plurality of peptides as described above, the plurality of functional peptides can be inserted in any order and fused with a ferritin monomer. Fusion can be achieved via an amide bond. Fusion may be achieved directly by an amide bond or indirectly by an amide bond mediated by a peptide (peptide linker) consisting of one amino acid residue or several (for example, 2 to 20, preferably 2 to 10, more preferably 2, 3, 4 or 5) amino acid residues. Since various peptide linkers are known, such peptide linkers can also be used in the present invention. Preferably, the length of the entire peptide inserted into the region described above is 20 amino acid residues or less.

When a peptide having a binding ability to a target material is used as the functional peptide, examples of the target material include a biological organic molecule, a material (for example, nickel, maltose, or glutathione) capable of interacting with a tag for protein purification (for example, a histidine tag, a maltose binding protein tag, or glutathione-S-transferase), and a labelling substance (for example, a radioactive substance, a fluorescent substance, or a dye). Ferritin is known to be taken up by transferrin receptor presenting cells (for example, L. Li et al. Proc Natl Acad Sci USA. 2010; 107 (8): 3505-10). When a peptide having a binding ability to a biological organic molecule is used as the functional peptide, ferritin can be delivered to cells and organs expressing a biological organic molecule to which ferritin is not bonded originally.

Examples of the biological organic molecule include a protein (for example, an oligopeptide or a polypeptide), a nucleic acid (for example, DNA or RNA, a nucleoside, a nucleotide, an oligonucleotide, or a polynucleotide), a carbohydrate (for example, a monosaccharide, an oligosaccharide, or a polysaccharide), and a lipid. The biological organic molecule may also be a cell surface antigen (for example, a cancer antigen, a cardiac disease marker, a diabetes marker, a neurological disease marker, an immune disease marker, an inflammatory marker, a hormone, or an infectious disease marker). The biological organic molecule may also be a disease antigen (for example, a cancer antigen, a cardiac disease marker, a diabetes marker, a neurological disease marker, an immune disease marker, an inflammatory marker, a hormone, or an infectious disease marker). As a peptide having a binding ability to such a biological organic molecule, various peptides have been reported. For example, various peptides such as a peptide having a binding ability to a protein (see, for example, F. Danhier et al., Mol. Pharmaceutics, 2012, vol. 9, No. 11, p. 2961, C-H. Wu et al., Sci. Transl. Med., 2015, vol. 7, No. 290, 290 ra 91., L. Vannucci et. al. Int. J. Nanomedicine. 2012, vol.7, p. 1489; J. Cutrera et al., Mol. Ther. 2011, vol. 19 (8), p. 1468, and R. Liu et al., Adv. Drug Deliv. Rev. 2017, vol. 110-111, p. 13), a peptide having a binding ability to a nucleic acid (see, for example, R. Tan et. al. Proc. Natl. Acad. Sci. USA, 1995, vol. 92, p. 5282, R. Tan et. al. Cell, 1993, vol. 73, p. 1031, and R. Talanian et. al. Biochemistry. 1992, vol. 31, p. 6871), a peptide having a binding ability to a carbohydrate (see, for example, K. Oldenburg et. al., Proc. Natl. Acad. Sci. USA, 1992, vol. 89, No. 12, p. 5393-5397, K. Yamamoto et. al., J. Biochem., 1992, vol. 111, p. 436, and A. Baimiev et. al., Mol. Biol. (Moscow), 2005, vol. 39, No. 1, p. 90), and a peptide having a binding ability to a lipid (see, for example, O. Kruse et. al., B Z. Naturforsch, 1995, vol. 50c, p. 380, O. Silva et. al., Sci. Rep., 2016, vol. 6, 27128, and A. Filoteo et. al., J. Biol. Chem., 1992, vol. 267, No. 17, p. 11800) have been reported.

When a protease-degradable peptide is used as the functional peptide, examples of the protease include a cysteine protease such as caspase or cathepsin (D. McIlwain1 et al., Cold Spring Harb Perspect Biol., 2013, vol. 5, a008656 and V. Stoka et al., IUBMB Life. 2005, vol. 57, No. 4-5 p. 347), collagenase (G. Lee et al., Eur J Pharm Biopharm., 2007, vol. 67, No. 3), p. 646), thrombin and a factor Xa (R. Jenny et al., Protein Expr. Purif., 2003, vol. 31, p. 1 and H. Xu et al., J. Virol., 2010, vol. 84, No. 2, p. 1076), and virus-derived proteases (C. Byrd et al., Drug Dev. Res., 2006, vol. 67, p. 501). Various protease-degradable peptides are known (for example, E. Lee et al., Adv. Funct. Mater., 2015, vol. 25, p. 1279; G. Lee et al., Eur J Pharm Biopharm., 2007, vol. 67, No. 3, p. 646; Y. Kang et al., Biomacromolecules, 2012, vol. 13, No. 12, p. 4057; R. Talanian et al., J. Biol. Chem., 1997, vol. 272, p. 9677; Jenny et al., Protein Expr. Purif., 2003, vol. 31, p. 1; and H. Xu et al., J. Virol., 2010, vol. 84, No. 2, p. 1076). In the present invention, various protease-degradable peptides can be used (for example, E. Lee et al., Adv. Funct. Mater., 2015, vol. 25, p. 1279; G. Lee et al., Eur J Pharm Biopharm., 2007, vol. 67, No. 3, p. 646; Y. Kang et al., Biomacromolecules, 2012, vol. 13, No. 12, p. 4057; R. Talanian et al., J. Biol. Chem., 1997, vol. 272, p. 9677; Jenny et al., Protein Expr. Purif., 2003, vol. 31, p. 1; and H. Xu et al., J. Virol., 2010, vol. 84, No. 2, p. 1076).

When a stabilized peptide is used as the functional peptide, various stabilized peptides can be used (for example, X. Meng et al., Nanoscale, 2011, vol.3, No.3, p. 977 and E. Falvo et al., Biomacromolecules, 2016, vol. 17, No. 2, p. 514).

When a cell penetrating peptide is used as the functional peptide, various cell penetrating peptides can be used (for example, Z. Guo et al. Biomed. Rep., 2016, vol. 4, No. 5, p. 528).

The functional peptide is preferably a peptide having a binding ability to a target material. The peptide having a binding ability to a target material is preferably a peptide having a binding ability to a biological organic molecule, and more preferably a peptide having a binding ability to a protein.

The human ferritin H chain may be modified in an N-terminal region thereof and/or a C-terminal region thereof. The N-terminal of an animal ferritin monomer such as a human ferritin monomer is exposed on a surface of a multimer, and a C-terminal thereof cannot be exposed on the surface. Therefore, although a peptide moiety added to the N-terminal of the animal ferritin monomer is exposed on a surface of a multimer and can interact with a target material present outside the multimer, a peptide moiety added to the C-terminal of the animal ferritin monomer is not exposed on the surface of the multimer and cannot interact with the target material present outside the multimer (for example, WO 2006/126595 A). However, it has been reported that the C-terminal of the animal ferritin monomer can be used for encapsulation of a drug into an internal cavity of a multimer by modifying an amino acid residue of the C-terminal (see, for example, Y. J. Kang, Biomacromolecules. 2012, vol. 13 (12), 4057).

In a certain embodiment, the human ferritin H chain may have a peptide moiety added to an N-terminal as modification in an N-terminal region of the human ferritin H chain. Examples of the peptide moiety to be added include a functional peptide as described above. Alternatively, examples of the peptide moiety to be added include a peptide component that improves solubility of a target protein (for example, Nus-tag), a peptide component that acts as a chaperone (for example, a trigger factor), a peptide component having other functions (for example, a full-length protein or a part thereof), and a linker. As the peptide moiety added to the N-terminal of the human ferritin H chain, a peptide that is the same as or different from a functional peptide inserted into a region between the second and third α-helices can be used, but a different peptide is preferably used from a viewpoint of, for example, achieving an interaction with a different target material. The peptide moiety added to the N-terminal of the human ferritin H chain is preferably a functional peptide as described above. The peptide moiety added to the N-terminal is preferably designed so as to contain an amino acid residue (for example, a methionine residue) corresponding to an initiation codon at the N-terminal. Such a design can promote translation of the human ferritin H chain.

In another certain embodiment, in the human ferritin H chain, as modification in the C-terminal region thereof, an amino acid residue in the C-terminal region may be replaced with a reactive amino acid residue, a reactive amino acid residue may be inserted into the C-terminal region, or a reactive amino acid residue or a peptide containing the reactive amino acid residue (a peptide consisting of, for example, 2 to 12, preferably 2 to 5 amino acid residues) may be added to the C-terminal. For example, for the human ferritin H chain, such a C-terminal region is a region consisting of amino acid residues at positions 175 to 183 (preferably positions 179 to 183). By such modification, a reactive amino acid residue and a certain substance (for example, a drugs or a labelling substance) can be caused to react, whereby the certain substance can be encapsulated in an internal cavity of a multimer via a covalent bond. Examples of such a reactive amino acid residue include a cysteine residue having a thiol group, a lysine residue having an amino group, an arginine residue, an asparagine residue, and a glutamine residue, and a cysteine residue is preferable. The modification of the C-terminal region of the fusion protein of the present invention is preferably addition of a reactive amino acid residue or a peptide containing the reactive amino acid residue to the C-terminal.

In a preferred embodiment, when the human ferritin H chain further contains a functional peptide, the functional peptide may be a human-derived peptide. When the functional peptide is a human-derived peptide, a risk of developing immunogenicity in humans can be reduced.

The human ferritin H chain contained in the modified ferritin of the present invention is preferably a natural human ferritin H chain optionally having a deletion of a methionine residue at position 1.

The modified ferritin of the present invention is a multimer containing a human ferritin H chain as described above. A human ferritin can form a 24-mer containing 24 ferritin monomers (H and L chains). Ferritin naturally occurring in humans is usually a hetero 24-mer, which is a mixture of H and L chains, and ferritin experimentally prepared is usually a homo 24-mer. This is because a burden to separately prepare ferritin of the H chain and ferritin of the L chain and to mix them is necessary in a case of preparing the hetero 24-mer in an experiment, and thus the homo 24-mer is prepared from a viewpoint of reducing the experimental burden. Therefore, the modified ferritin of the present invention may be a homo 24-mer containing only the human ferritin H chain or a hetero 24-mer containing the human ferritin H and L chains. In this case, the human ferritin L chain may contain a mutation (for example, a mutation to further contain a functional peptide, and modifications of an N-terminal and a C-terminal) as described above in the human ferritin H chain. The homo 24-mer containing only the human ferritin H chain is preferable from a viewpoint of simple preparation.

The multimer containing the human ferritin H chain can be obtained by producing, using a host cell containing a polynucleotide encoding the human ferritin H chain, the human ferritin H chain in the host cell. Examples of the host cell for producing the human ferritin H chain include cells derived from an animal, an insect, a plant, and a microorganism. As the animal, a mammal or a bird (for example, a hen and a cock) is preferable, and a mammal is more preferable. Examples of the mammal include a primate (for example, a human, a monkey, or a chimpanzee), a rodent (for example, a mouse, a rat, a hamster, a guinea pig, or a rabbit), and a livestock or working mammal (for example, a bovine, a pig, a sheep, a goat, or a horse).

In a preferred embodiment, the host cell is a human cell or a cell that is widely used for production of a human protein (for example, a Chinese hamster ovary (CHO) cell or a human embryonic kidney-derived HEK 293 cell). Such a host cell is preferably used from a viewpoint of clinical application to humans.

In another preferred embodiment, the host cell is a microorganism. Such a host cell may be used from a viewpoint of, for example, mass production of a fusion protein. Examples of the microorganism include a bacterium and a fungus. As the bacterium, any bacterium used as a host cell can be used, and examples thereof include a bacterium belonging to the genus Bacillus [for example, Bacillus subtilis], a bacterium belonging to the genus Corynebacterium [(for example, Corynebacterium glutamicum)], a bacterium belonging to the genus Escherichia [for example, Escherichia coli], and a bacterium belonging to the genus Pantoea (for example, Pantoea ananatis). As the fungus, any fungus used as a host cell can be used, and examples thereof include a fungus belonging to the genus Saccharomyces [for example, Saccharomyces cerevisiae] and a fungus belonging to the genus Schizosaccharomyces [for example, Schizosaccharomyces pombe]. Alternatively, a filamentous fungus may be used as the microorganism. Examples of the filamentous fungus include bacteria belonging to the genus Acremonium/Talaromyces, the genus Trichoderma, the genus Aspergillus, the genus Neurospora, the genus Fusarium, the genus Chrysosporium, the genus Humicola, the genus Emericella, and the genus Hypocrea.

The host cell can be designed so as to contain, in addition to a polynucleotide encoding the human ferritin H chain, an expression unit containing a promoter operably linked to the polynucleotide. The term "expression unit" refers to a unit that contains a certain polynucleotide to be expressed as a protein and a promoter operably linked thereto and enables transcription of the polynucleotide and consequently production of a protein encoded by the polynucleotide. The expression unit may further contain an element such as a terminator, a ribosome binding site, or a drug-resistant gene. The expression unit may be DNA or RNA, and is preferably DNA. The expression unit can be contained in a genomic region (for example, a natural genomic region that is a natural locus in which a polynucleotide encoding the protein is uniquely present, or a non-natural genomic region that is not the natural locus) or a non-genomic region (for example, in a cytoplasm) in a microorganism (host cell). The expression unit may be contained in a genomic region at one or more (for example, 1, 2, 3, 4, or 5) different positions. Examples of a specific form of the expression unit contained in the non-genomic region include a plasmid, a viral vector, a phage, and an artificial chromosome.

As long as the modified ferritin of the present invention contains one or more human ferritin H chains each containing a modifying group covalently bonded specifically to a cysteine residue at position 91 and/or position 103, the modified ferritin may further contain a human ferritin H chain not containing the modifying group. Such a modified ferritin can be prepared by reacting ferritin with a reactive substance (for example, a thiol modifying reagent and a functional substance) at a low molar ratio. Such a modified ferritin can also be prepared by separately preparing a 24-mer containing a human ferritin H chain containing a modifying group covalently bonded specifically to a cysteine residue at position 91 and/or position 103 and a 24-mer containing a human ferritin H chain not containing the modifying group, and then dissociating and reassociating these 24-mers in a solution containing these 24-mers at a certain molar ratio. Ferritin is relatively easily capable of dissociation and association, and therefore is in a form of a 24-mer or a monomer, or in a form in which the 24-mer and the monomer coexist depending on conditions of a solvent or the like containing the ferritin. Therefore, by appropriately adjusting conditions of a solvent or the like containing ferritin, the 24-mer can be dissociated into monomers, and the dissociated monomers can be reassociated to the 24-mer. Such dissociation and reassociation are used in encapsulating various substances in an internal cavity of ferritin (see, for example, WO 2020/090708; CN 106110333 A; Journal of Controlled Release 196 (2014) 184-196; Biomacromolecules 2016, 17,514-522; and Proc Natl Acad Sci USA. 2014 111 (41): 14900-5). When the modified ferritin of the present invention further contains a human ferritin H chain not containing a modifying group, molar ratios of the human ferritin H chain containing a modifying group and the human ferritin H chain not containing a modifying group in the modified ferritin of the present invention can be controlled according to a molar ratio of a reactive substance to ferritin or molar ratios of the human ferritin H chain containing a modifying group and the human ferritin H chain not containing a modifying group before reassociation in a solution. The modified ferritin of the present invention is preferably a 24-mer containing 24 human ferritin H chains each containing a modifying group covalently bonded specifically to a cysteine residue at position 91 and/or position 103.

The human ferritin H chain contained in the modified ferritin of the present invention contains a modifying group covalently bonded specifically to a cysteine residue at position 91 and/or position 103. A "specific" covalent bond in the modified ferritin of the present invention means that selectivity of the covalent bond to the cysteine residue at position 91 and/or position 103 (more strictly, a sulfur atom in a side chain in the cysteine residue) is significantly higher than that to another amino acid residue. Preferably, the "specific" covalent bond in the modified ferritin of the present invention is one that is covalently bonded only to the cysteine residue at position 91 and/or position 103 and is not covalently bonded to another amino acid residue.

The amino acid residue modified in the present invention is a cysteine residue at position 91 and/or position 103 present in the human ferritin H chain. In a human ferritin, the cysteine residues at positions 91 and 103 are close to each other. The modified ferritin of the present invention can contain one or two modifying groups per human ferritin H chain at such a position. Therefore, the present invention can provide a modified ferritin containing a human ferritin H chain containing a modifying group of either (A) or (B) below:
(A) a human ferritin H chain containing one or two modifying groups which are covalently bonded specifically to one or two sulfur atoms in a side chain of one or both cysteine residues at positions 91 and 103; or
(B) a human ferritin H chain containing one modifying group bridging two sulfur atoms in side chains of cysteine residues at positions 91 and 103 by a specific covalent bond.

A modified ferritin containing such a human ferritin H chain can be prepared by setting a molar ratio between a human ferritin and a thiol modifying reagent or a functional substance described later (human ferritin : thiol modifying reagent or functional substance) to be, for example, in a range of 1 : 1 to 1 : 30, preferably 1 : 1 to 1 : 20, more preferably 1 : 1 to 1 : 15.

The modified ferritin of the present invention can preferably contain 24 or 48 modifying groups. This is because the modified ferritin of the present invention is a 24-mer which can contain 1 or 2 modifying groups per human ferritin H chain at cysteine residues at positions 91 and 103.

The modified ferritin of the present invention can also contain two or more types of modifying groups (for example, modifying groups containing two or more types of reactive groups or functional substances). The present invention can highly control the number of modifying groups introduced into the modified ferritin. For example, by introducing 24 first modifying groups into ferritin and then introducing 24 second modifying groups into the obtained modified ferritin, a modified ferritin containing two or more types of modifying groups in which the number of introduced groups is highly controlled can be obtained. This is because the modified ferritin of the present invention is a 24-mer which can contain 1 or 2 modifying groups per human ferritin H chain at cysteine residues at positions 91 and 103.

In one embodiment, the modified ferritin of the present invention may be a reactive group-added ferritin wherein a modifying group contains a reactive group.

That is, in this case,
provided is a reactive group-added ferritin containing a human ferritin H chain, in which
the human ferritin H chain contains a reactive group-containing modifying group covalently bonded specifically to a cysteine residue at position 91 and/or position 103.

For example, such a reactive group-added ferritin is useful as a synthetic intermediate of a functional substance-added ferritin.

The reactive group-added ferritin can be produced by reacting a human ferritin with a thiol modifying reagent. Ferritin used in the reaction may be in either a form of a 24-mer or a form of a monomer, but is preferably in a form of a 24-mer. The thiol modifying reagent used in the present invention is a reactive substance containing at least one reaction site for a thiol group, and has an ability to add a modifying group containing a reactive group to a human ferritin by a reaction. As the thiol modifying reagent, any reagent capable of catalyzing such a reaction can be used. The thiol modifying reagent preferably contains one or more partial structures selected from the group consisting of a maleimide moiety, a benzyl halide moiety, an α-haloamide moiety, an α-haloketone moiety, an alkene moiety, an alkyne moiety, a fluoroaryl moiety, a nitroaryl moiety, a methylsulfonyloxadiazole moiety, and a disulfide moiety from a viewpoint of improving reaction efficiency.

The reactive group can be appropriately set so as to react with the functional substance. Therefore, the reactive group is not particularly limited as long as the reactive group contains a moiety capable of reacting with a certain functional group in the functional substance (the functional substance may be derivatized so to have a certain functional group capable of reacting with the reactive group). The number of reactive groups contained in the modifying group is one or more, for example 1 to 5, preferably 1 to 3, more preferably 1 or 2, and still more preferably 1. When a plurality of reactive groups is contained in the modifying group, the plurality of reactive groups may be of the same type or different types. For example, when a reaction with a plurality of functional substances of the same type is desired, reactive groups of the same type are preferable from a viewpoint of adopting a simple structure or the like. Meanwhile, when a reaction with a plurality of functional substances of different types is desired, reactive groups of different types are preferable from a viewpoint of ensuring reaction selectivity or the like.

In a certain embodiment, the reactive group may be a bioorthogonal functional group for a protein. In such a case, a reaction between the reactive group-added ferritin and the functional substance can be promoted while a reaction between molecules of the reactive group-added ferritin is suppressed. The bioorthogonal functional group refers to a group having such reaction selectivity that the bioorthogonal functional group cannot react with a biological component (for example, an amino acid, a nucleic acid, a lipid, a sugar, or a phosphoric acid) or hardly reacts with the biological component but easily reacts with a component other than the biological component. The bioorthogonal functional group is well known in the technical field concerned (see, for example, Sharpless K. B. et al., Angew. Chem. Int. Ed. 40, 2004 (2015); Bertozzi C. R. et al., Science 291, 2357 (2001); and Bertozzi C. R. et al., Nature Chemical Biology 1, 13 (2005)). Therefore, the bioorthogonal functional group for a protein means a group having such reaction selectivity that the bioorthogonal functional group cannot react with a side chain of 20 types of natural amino acid residues constituting the protein or hardly reacts with the side chain but easily reacts with a target other than the side chain. The 20 types of natural amino acids constituting the protein are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Among these 20 types of natural amino acids, glycine, which has no side chain (that is, which has a hydrogen atom as a side chain), and alanine, isoleucine, leucine, phenylalanine, and valine, which each have a hydrocarbon group as a side chain (that is, which each contain no hetero atom selected from the group consisting of a sulfur atom, a nitrogen atom, and an oxygen atom in a side chain thereof) are inactive to a normal reaction. Therefore, the bioorthogonal functional group for a protein is a functional group incapable of reacting with, in addition to side chains of these amino acids having side chains inactive to a normal reaction, side chains of asparagine, glutamine, methionine, proline, serine, threonine, tryptophan, tyrosine, aspartic acid, glutamic acid, arginine, histidine, and lysin, and optionally a side chain of cysteine (for example, when a cysteine residue at position 91 and/or position 103 of the human ferritin H chain is not sufficiently modified).

Examples of the bioorthogonal functional group for a protein include a group containing one or more partial structures selected from the group consisting of a maleimide moiety, an azide moiety, a ketone moiety, an aldehyde moiety, a thiol moiety, an alkene moiety (in other words, it is only required to have a vinyl (ethenyl) moiety or a vinylene (ethenylene) moiety which is a minimum unit having a carbon-carbon double bond, hereinafter the same), an alkyne residue (in other words, it is only required to have an ethynyl moiety or an ethynylene moiety which is a minimum unit having a carbon-carbon triple bond, hereinafter the same), a halogen moiety (for example, a fluorinated moiety, a chlorinated moiety, a brominated moiety, or an iodinated moiety), a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety (for example, a carbonyl moiety having a fluorine atom, a chlorine atom, a bromine atom, or an iodine atom at an α-position), an isonitrile moiety, a sydnone moiety, and a selenium moiety. The protein includes a protein that can contain a free thiol in a side chain of a cysteine residue (for example, a protein other than an antibody) and a protein that cannot contain a free thiol (for example, an antibody, a protein not containing a cysteine residue, or a ferritin containing a human ferritin H chain in which a cysteine residue at position 91 and/or position 103 is sufficiently modified as provided in the present invention). In the protein that cannot contain a free thiol, a thiol functions as a bioorthogonal functional group. Therefore, in the present invention, in principle, the bioorthogonal functional group for a protein contains a thiol moiety. However, when the cysteine residue at position 91 and/or position 103 is not sufficiently modified (for example, when the average modification number of a cysteine residue per human ferritin H chain is 1), the bioorthogonal functional group for a protein does not contain a thiol moiety.

The reactive group can also be set so as to specifically react with a certain functional group in a functional substance that can react with the reactive group later depending on the type of the functional substance (for example, a peptide, a protein, a nucleic acid, a lipid, or a low molecular organic compound).

The reactive group only needs to be able to react with the functional substance. Therefore, the reactive group may have a substituent as long as the reactive group contains a partial structure capable of reacting with a certain functional group in the functional substance. For example, in order to simplify a structure of a moiety capable of reacting with a certain functional group in the functional substance, the partial structure preferably has no substituent. However, when there is a replaceable hydrogen atom in a moiety capable of reacting with a certain functional group in the functional substance, the partial structure preferably has a substituent. The number of such substituents varies depending on a factor such as the number of replaceable hydrogen atoms, and is, for example, 1 to 5, preferably 1 to 3, more preferably 1 or 2, and still more preferably 1. Examples of such a substituent include a monovalent hydrocarbon group, a monovalent heterocyclic group, a halogen atom (for example, a fluorine atom, a chlorine atom, a bromine atom, or an iodine atom), a hydroxyl group, a nitro group, a sulfuric acid group, a sulfonic acid group, a cyano group, and a combination of two or more (for example, 2 to 6, preferably 2 to 5, more preferably 2 to 4, still more preferably 2 or 3, particularly preferably 2) thereof.

Examples of the monovalent hydrocarbon group include a monovalent chain hydrocarbon group, a monovalent alicyclic hydrocarbon group, and a monovalent aromatic hydrocarbon group. The monovalent chain hydrocarbon group means a hydrocarbon group containing only a chain structure and does not contain any cyclic structure in a main chain thereof. Note that the chain structure may be linear or branched. Examples of the monovalent chain hydrocarbon group include an alkyl, an alkenyl, and an alkynyl. The alkyl, alkenyl, and alkynyl may be linear or branched.

The alkyl is preferably a C₁₋₁₂ alkyl, more preferably a C₁₋₆ alkyl, and still more preferably a C₁₋₄ alkyl. The number of carbon atoms does not include the number of carbon atoms of a substituent. Examples of the C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably a C₂₋₁₂ alkenyl, more preferably a C₂₋₆ alkenyl, and still more preferably a C₂₋₄ alkenyl. The number of carbon atoms does not include the number of carbon atoms of a substituent. Examples of the C₂₋₁₂ alkenyl include vinyl, propenyl, and n-butenyl.

The alkynyl is preferably a C₂₋₁₂ alkynyl, more preferably a C₂₋₆ alkynyl, and still more preferably a C₂₋₄ alkynyl. The number of carbon atoms does not include the number of carbon atoms of a substituent. Examples of the C₂₋₁₂ alkynyl include ethynyl, propynyl, and n-butynyl.

The monovalent chain hydrocarbon group is preferably an alkyl.

The monovalent alicyclic hydrocarbon group means a hydrocarbon group containing only an alicyclic hydrocarbon as a cyclic structure and not containing any aromatic ring, in which the alicyclic hydrocarbon may be monocyclic or polycyclic. Note that the monovalent alicyclic hydrocarbon group is not necessarily required to contain only an alicyclic hydrocarbon but may contain a chain structure in a part thereof. Examples of the monovalent alicyclic hydrocarbon group include cycloalkyl, cycloalkenyl, and cycloalkynyl, which may be monocyclic or polycyclic.

The cycloalkyl is preferably a C₃₋₁₂ cycloalkyl, more preferably a C₃₋₆ cycloalkyl, and still more preferably a C₅₋₆ cycloalkyl. The number of carbon atoms does not include the number of carbon atoms of a substituent. Examples of the C₃₋₁₂ cycloalkyl include cyclopropyl, cyclobutyl, cyclopentyl, and cyclohexyl.

The cycloalkenyl is preferably a C₃₋₁₂ cycloalkenyl, more preferably a C₃₋₆ cycloalkenyl, and still more preferably a C₅₋₆ cycloalkenyl. The number of carbon atoms does not include the number of carbon atoms of a substituent. Examples of the C₃₋₁₂ cycloalkenyl include cyclopropenyl, cyclobutenyl, cyclopentenyl, and cyclohexenyl.

The cycloalkynyl is preferably a C₃₋₁₂ cycloalkynyl, more preferably a C₃₋₆ cycloalkynyl, and still more preferably a C₅₋₆ cycloalkynyl. The number of carbon atoms does not include the number of carbon atoms of a substituent. Examples of the C₃₋₁₂ cycloalkynyl include cyclopropynyl, cyclobutynyl, cyclopentynyl, and cyclohexynyl.

The monovalent alicyclic hydrocarbon group is preferably a cycloalkyl.

The monovalent aromatic hydrocarbon group means a hydrocarbon group containing an aromatic cyclic structure. Note that the monovalent aromatic hydrocarbon group is not necessarily required to contain only an aromatic ring and may contain a chain structure or alicyclic hydrocarbon in a part thereof, in which the aromatic ring may be monocyclic or polycyclic. The monovalent aromatic hydrocarbon group is preferably a C₆₋₁₂ aryl, more preferably a C₆₋₁₀ aryl, and still more preferably a C₆ aryl. The number of carbon atoms does not include the number of carbon atoms of a substituent. Examples of the C₆₋₁₂ aryl include phenyl and naphthyl.

The monovalent aromatic hydrocarbon group is preferably phenyl.

Among these groups, the monovalent hydrocarbon group is preferably an alkyl, a cycloalkyl, or an aryl, and more preferably an alkyl.

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably contains one or more selected from the group consisting of an oxygen atom, a sulfur atom, a nitrogen atom, a phosphorus atom, a boron atom, and a silicon atom and more preferably contains one or more selected from the group consisting of an oxygen atom, a sulfur atom, and a nitrogen atom as a hetero atom constituting the heterocyclic group.

The monovalent aromatic heterocyclic group is preferably a C₁₋₁₅ aromatic heterocyclic group, more preferably a C₁₋₉ aromatic heterocyclic group, and still more preferably a C₁₋₆ aromatic heterocyclic group. The number of carbon atoms does not include the number of carbon atoms of a substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and still more preferably a C₂₋₆ nonaromatic heterocyclic group. The number of carbon atoms does not include the number of carbon atoms of a substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

A reaction for producing a reactive group-added ferritin can be appropriately performed under a condition incapable of causing denaturation or decomposition (for example, cleavage of an amide bond) of ferritin (a mild condition). For example, such a reaction can be performed in an appropriate reaction system, for example, in a buffer under a temperature condition that cannot cause denaturation or decomposition of ferritin (for example, about 15 to 60°C, preferably 15 to 45°C). The pH of the buffer is, for example, 3.0 to 12.0, preferably 5.0 to 9.0, and more preferably 6.0 to 8.0. The buffer may contain a component such as a catalyst. Reaction time is for example, one minute to 20 hours, preferably 10 minutes to 15 hours, more preferably 20 minutes to 10 hours, and still more preferably 30 minutes to eight hours.

In another embodiment, the modified ferritin of the present invention may be a functional substance-added ferritin in which a modifying group contains a functional substance.

That is, in this case,
provided is a functional substance-added ferritin containing a human ferritin H chain, in which
the human ferritin H chain contains a functional substance-containing modifying group covalently bonded specifically to a cysteine residue at position 91 and/or position 103.

For example, such a functional substance-added ferritin is useful for prevention, treatment, or diagnosis of a specific disease in a human.

The functional substance is not particularly limited as long as the functional substance is a substance that imparts any function to ferritin, and examples thereof include a drug, a labelling substance, and a stabilizer. The functional substance is preferably a drug or a labelling substance. The functional substance may be a single functional substance or a substance in which two or more functional substances are linked to each other.

The drug may be a drug to any disease. Examples of such a disease include cancer (for example, a lung cancer, a stomach cancer, a colon cancer, a pancreatic cancer, a renal cancer, a liver cancer, a thyroid cancer, a prostatic cancer, a bladder cancer, an ovarian cancer, a uterine cancer, a bone cancer, a skin cancer, a brain tumor, or melanoma), an autoimmune disease and an inflammatory disease (for example, an allergic disease, articular rheumatism, or systemic lupus erythematosus), a brain or nerve disease (for example, cerebral infarction, Alzheimer's disease, Parkinson disease, or amyotrophic lateral sclerosis), an infectious disease (for example, a microbial infectious disease or a viral infectious disease), a hereditary rare disease (for example, hereditary spherocytosis or nondystrophic myotonia), an eye disease (for example, age-related macular degeneration, diabetic retinopathy, or retinitis pigmentosa), a diseases in the bone and orthopedic field (for example, osteoarthritis), a blood disease (for example, leukosis or purpura), and other diseases (for example, diabetes, a metabolic disease such as hyperlipidemia, a liver disease, a renal disease, a lung disease, a circulatory system disease, or a digestive system disease). The drug may be a prophylactic or therapeutic agent for a disease, or a relief agent for a side effect.

More specifically, the drug is an anti-cancer agent. Examples of the anti-cancer agent include a chemotherapeutic agent, a toxin, a radioisotope, and substances containing these. Examples of the chemotherapeutic agent include a DNA injuring agent, an antimetabolite, an enzyme inhibitor, a DNA intercalating agent, a DNA cleaving agent, a topoisomerase inhibitor, a DNA binding inhibitor, a tubulin binding inhibitor, a cytotoxic nucleoside, and a platinum compound. Examples of the toxin include a bacteriotoxin (for example, a diphtheria toxin) and a phytotoxin (for example, ricin). Examples of the radioisotope include a radioisotope of a hydrogen atom (for example, ³H), a radioisotope of a carbon atom (for example, ¹⁴C), a radioisotope of a phosphorous atom (for example, ³²P), a radioisotope of a sulfur atom (for example, 35^{s}), a radioisotope of yttrium (for example, ⁹⁰Y), a radioisotope of technetium (for example, ^{99m}Tc), a radioisotope of indium (for example, ¹¹¹In), a radioisotope of an iodide atom (for example, ¹²³I, ¹²⁵I, ¹²⁹I, and ¹³¹I), a radioisotope of samarium (for example, ¹⁵³Sm), a radioisotope of rhenium (for example, ¹⁸⁶Re), a radioisotope of astatine (for example, ²¹¹At), and a radioisotope of bismuth (for example, ²¹²Bi). More specific examples of the drug include auristatin (MMAE or MMAF), maytansine (DM1 or DM4), PBD (pyrrolobenzodiazepine), IGN, a camptothecin analog, calicheamicin, duocarmycin, eribulin, anthracycline, dmDNA31, and tubricin.

A targeting substance is an affinity substance for a target (for example, a tissue, a cell, or a substance). Examples of the targeting substance include an antibody to a target substance or a fragment thereof having a binding ability to a target substance, a ligand having a binding ability to a receptor, a protein capable of forming a complex (for example, an adaptor protein), a protein having a binding ability to a sugar chain (lectin), a sugar chain having a binding ability to a protein, and a nucleic acid having a binding ability to a complementary sequence (for example, a natural nucleic acid such as DNA or RNA, or an artificial nucleic acid).

The labelling substance is a substance that makes detection of a target (for example, a tissue, a cell, or a substance) possible. Examples of the labelling substance include an enzyme (for example, peroxidase, alkaline phosphatase, luciferase, or β-galactosidase), an affinity substance (for example, streptavidin, biotin, digoxigenin, or aptamer), a fluorescent substance (for example, fluorescein, fluorescein isothiocyanate, rhodamine, green-fluorescent protein, or red-fluorescent protein), a luminescent substance (for example, luciferin, aequorin, acridinium ester, tris(2,2'-bipyridyl) ruthenium, or luminol), a radioisotope (for example, those described above), and substances containing these.

The stabilizer is a substance that makes stabilization of ferritin possible. Examples of the stabilizer include a diol, glycerin, a nonionic surfactant, an anionic surfactant, a natural surfactant, a saccharide, and a polyol.

The functional substance may also be a peptide, a protein (for example, an antibody), a nucleic acid (for example, DNA, RNA, or an artificial nucleic acid), a low molecular organic compound (for example, a low molecular organic compound described below), a chelator, a sugar chain, a lipid, a high molecular polymer, or a metal (for example, gold).

The functional substance-added ferritin can be produced by reacting a human ferritin with a functional substance. The functional substance used in such a reaction is a reactive substance containing at least one reaction site for a thiol group, and has an ability to add a modifying group containing the functional substance to a human ferritin by a reaction. As the functional substance used in such a reaction, any functional substance having a functional group that easily reacts with a thiol group can be used. Alternatively, when the functional substance does not have a functional group that easily reacts with a thiol group, a functional substance derivatized so as to have such a functional group can be used. The functional substance (which may be derivatized) preferably contains one or more partial structures selected from the group consisting of a maleimide moiety, a benzyl halide moiety, an α-haloamide moiety, an α-haloketone moiety, an alkene moiety, an alkyne moiety, a fluoroaryl moiety, a nitroaryl moiety, a methylsulfonyloxadiazole moiety, and a disulfide moiety from a viewpoint of improving reaction efficiency.

The functional substance-added ferritin can also be produced by reacting a human ferritin with a thiol modifying reagent, and then further reacting a reactive group-added ferritin obtained by the reaction with a functional substance. The step of reacting the human ferritin with the thiol modifying reagent can be performed as described above. The functional substance used in the step of further reacting the reactive group-added ferritin with the functional substance is a reactive substance containing at least one reaction site for a reactive group in the reactive group-added ferritin, and has an ability to add a modifying group containing the functional substance to a human ferritin by a reaction. As the functional substance used in such a reaction, any functional substance having a functional group that easily reacts with a reactive group can be used. Alternatively, when the functional substance does not have a functional group that easily reacts with a reactive group, a functional substance derivatized so as to have such a functional group can be used. Therefore, the functional group in the functional substance used in the reaction can be appropriately determined depending on the type of reactive group.

Derivatization of the functional substance is common technical knowledge in the field concerned (for example, WO 2004/010957 A, US 2006/0074008 A, and US 2005/0238649 A). Derivatization may be performed using the cross-linking agent described above, for example. Alternatively, derivatization may be performed using a specific linker having a desired functional group. Such a linker may be able to separate the functional substance and the antibody from each other through cleavage of the linker in an appropriate environment (for example, intracellular or extracellular), for example. Examples of such a linker include a peptidyl linker decomposed by a specific protease [for example, an intracellular protease (for example, a protease present in lysosome or endosome) or an extracellular protease (for example, a secretory protease)] (for example, United States Patent No. 6,214,345 and Dubowchik et al., Pharm. Therapeutics 83: 67-123 (1999)), and a linker capable of being cleaved at a local acidic site present in a living body (for example, Unites States Patent Nos. 5,622,929, 5,122,368, and 5,824,805). The linker may be self-immolative (for example, WO 02/083180 A, WO 04/043493 A, and WO 05/112919 A). In the present invention, the derivatized functional substance can also be referred to simply as the "functional substance."

In a certain embodiment, the functional substance may be a functional substance having a functional group that easily reacts with a reactive group that is a bioorthogonal functional group for a protein, or a functional substance derivatized so as to have a functional group that easily reacts with the reactive group. The function group that easily reacts with the bioorthogonal functional group can vary depending on a specific type of bioorthogonal functional group. A person skilled in the art can select an appropriate functional group as the functional group that easily reacts with the bioorthogonal functional group appropriately (for example, Boutureira et al., Chem. Rev., 2015, 115, 2174-2195). Examples of the functional group that easily reacts with the bioorthogonal functional group include, but are not limited to, a maleimide group and a disulfide group when the bioorthogonal functional group corresponds to a thiol moiety, an azide group when the bioorthogonal functional group corresponds to an alkyne moiety, and a hydrazine group when the bioorthogonal functional group corresponds to an aldehyde moiety or a ketone moiety.

A reaction for producing the functional substance-added ferritin can be appropriately performed under a condition incapable of causing denaturation or decomposition (for example, cleavage of an amide bond) of ferritin (a mild condition). For example, such a reaction can be performed in an appropriate reaction system, for example, in a buffer under a temperature condition that cannot cause denaturation or decomposition of ferritin (for example, about 15 to 60°C, preferably 15 to 45°C). The pH of the buffer is, for example, 3.0 to 12.0, preferably 5.0 to 9.0, and more preferably 6.0 to 8.0. The buffer may contain a component such as a catalyst. Reaction time is for example, one minute to 20 hours, preferably 10 minutes to 15 hours, more preferably 20 minutes to 10 hours, and still more preferably 30 minutes to eight hours.

The modified ferritin of the present invention may have a substance in an internal cavity thereof. A substance can be encapsulated in the internal cavity of the modified ferritin by using a property of ferritin that can incorporate a substance. A human ferritin forms a cage-like structure having an internal cavity having an outer diameter of about 12 nm (inner diameter: 7 nm). Therefore, the size of the substance can be a size that allows the substance to be encapsulated in such an internal cavity. It has been reported that various inorganic and organic substances can be encapsulated in an internal cavity of ferritin (see, for example, WO 2020/090708; CN 106110333 A; Journal of Controlled Release 196 (2014) 184-196; Biomacromolecules 2016, 17,514-522; and Proc Natl Acad Sci USA. 2014 111 (41): 14900-5). The substance can be encapsulated in the internal cavity of the modified ferritin before and after ferritin is subjected to a modification reaction. The substance is preferably a low molecular organic compound. The low molecular organic compound refers to an organic compound having a molecular weight of 1,500 or lower. The low molecular organic compound is a natural compound or a synthesized compound. The molecular weight of the low molecular organic compound may be 1,200 or lower, 1,000 or lower, 900 or lower, 800 or lower, 700 or lower, 600 or lower, 500 or lower, 400 or lower, or 300 or lower. The molecular weight of the low molecular organic compound may be 30 or higher, 40 or higher, or 50 or higher. The low molecular organic compound may be such a drug or labelling substance as described above. Examples of the low molecular organic compound include an amino acid, an oligopeptide, a vitamin, a nucleoside, a nucleotide, an oligonucleotide, a monosaccharide, an oligosaccharide, a lipid, a fatty acid, and salts thereof.

The modified ferritin of the present invention may be provided as a set of a plurality of different types of modified ferritins containing a plurality of different types (for example, two, three, or four types) of substances when the modified ferritin has a substance in an internal cavity thereof. For example, when the modified ferritin of the present invention is provided as a set of two types of modified ferritins having two types of substances, such a set can be obtained by combining a first modified ferritin having a first substance and a second modified ferritin having a second substance different from the first substance, the first modified ferritin and the second modified ferritin being prepared separately.

Confirmation of production of the modified ferritin (for example, a reactive group-added ferritin or a functional substance-added ferritin), which depends on the type and molecular weight of a modifying group thereof, can be performed, for example, by electrophoresis, chromatography (for example, gel permutation chromatography, ion-exchange chromatography, reversed phase column chromatography, or HPLC), and/or mass spectrometry. Confirmation of position selectivity can be performed, for example, by peptide mapping. Peptide mapping can be performed, for example, by protease (for example, trypsin or chymotrypsin) treatment and mass spectrometry. For the protease, an endoprotease is preferred. Examples of such an endoprotease include trypsin, chymotrypsin, Glu-C, Lys-N, Lys-C, and Asp-N. The modified ferritin can be purified appropriately by any method such as chromatography (for example, the pieces of chromatography described above and affinity chromatography).

### Examples

Hereinafter, the present invention will be described in more detail by Examples, but the present invention is not limited to the Examples.

### Example 1: Preparation of human ferritin H chain

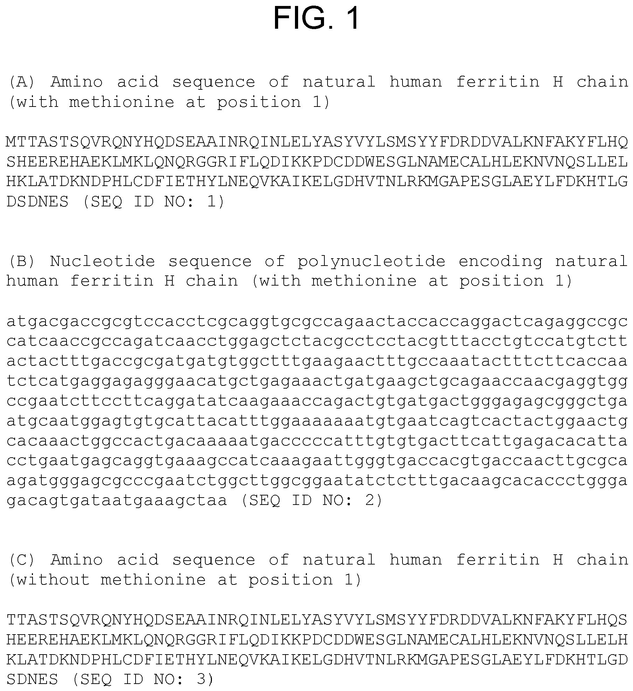

DNA (SEQ ID NO: 2) encoding a human ferritin H chain (FTH (SEQ ID NO: 1)) was totally synthesized. When an expression plasmid of this DNA is introduced into E. coli to express the human ferritin H chain in E. coli, methionine at an initiation codon becomes N-formylmethionine and is removed by post-translational modification. Therefore, the human ferritin H chain defined by the amino acid sequence of SEQ ID NO: 3 from which a methionine residue at position 1 in the amino acid sequence of SEQ ID NO: 1 has been removed is obtained (FIG. 1). Hereinafter, the position of an amino acid residue of a human ferritin is specified based on a typical natural human ferritin H chain defined by the amino acid sequence of SEQ ID NO: 1. If necessary, the position of the amino acid sequence of SEQ ID NO: 3 may also be described.

PCR was performed using synthetic DNA encoding a human ferritin H chain as a template and using 5'-GAAGGAGATATACATATGACGACCGCGTCCACCTCG-3' (SEQ ID NO: 4) and 5'-CTCGAATTCGGATCCTTAGCTTTCATTATCACTGTC-3' (SEQ ID NO: 5) as primers. In addition, PCR was performed using pET20 (Merck) as a template and using 5'-TTTCATATGTATATCTCCTTCTTAAAGTTAAAC-3' (SEQ ID NO. 6) and 5'-TTTGGATCCGAATTCGAGCTCCGTCG-3' (SEQ ID NO. 7) as primers. Each of the obtained PCR products was purified with a Wizard DNA Clean-Up System (Promega Corporation), and then subjected to In-Fusion enzyme treatment with In-Fusion HD Cloning Kit (Takara Bio Inc.) at 50°C for 15 minutes, thereby constructing an expression plasmid (pET20-FTH) on which a gene encoding FTH was mounted.

Subsequently, Escherichia coli BL21 (DE3) into which the constructed pET20-FTH was introduced was cultured in a flask containing 100 ml of LB medium (containing 10 g/l Bacto-typtone, 5 g/l Bacto-yeast extract, 5 g/l NaCl, and 100 mg/l ampicillin) at 37°C for 24 hours. The obtained bacterial cells were ultrasonically disrupted, and then the supernatant was heated at 60°C for 20 minutes. After heating, the supernatant obtained by cooling and centrifugation was injected into a HiPerp Q HP column (Cytiva) equilibrated with a 50 mM tris-HCl buffer (pH 8.0), and subjected to a salt concentration gradient with a 50 mM tris-HCl buffer (pH 8.0) containing 0 mM to 500 mM NaCl, thereby separating and purifying a target protein due to a difference in surface charge. A solvent of a solution containing the protein was replaced with a 10 mM tris-HCl buffer (pH 8.0) by centrifugal ultrafiltration using Vivaspn 20-100K (Cytiva). The solution was injected into a HiPrep 26/60 Sephacryl S-300 HR column (Cytiva) equilibrated with a 10 mM tris-HCl buffer (pH 8.0), and FTH was separated and purified. A solution containing the FTH was concentrated by centrifugal ultrafiltration using Vivaspn 20-100K (Cytiva), and the concentration of a protein contained was determined by a protein assay CBB solution (Nacalai Tesque, Inc.) using bovine albumin as a standard. As a result, 1 ml of a solution containing 5 mg/ml FTH could be obtained per 100 ml of the culture solution.

### Example 2: Specific modification of human ferritin H chain with ethyl maleimide and ESI-TOFMS analysis

### (2-1) Specific modification of human ferritin H chain with ethyl maleimide and ESI-TOFMS analysis

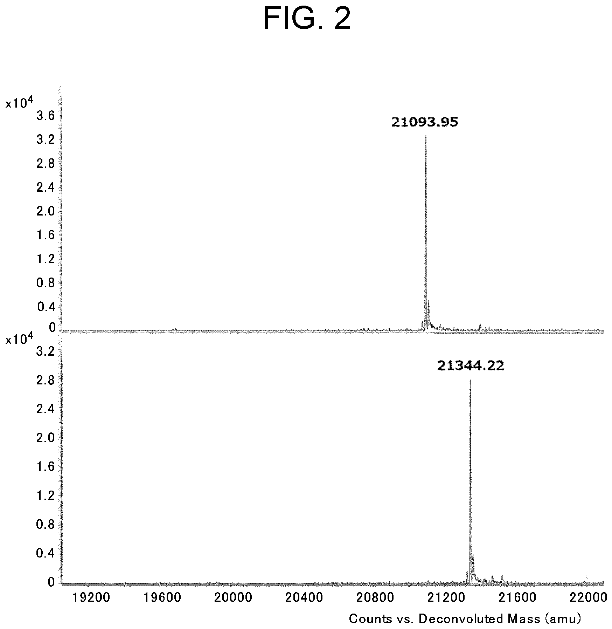

The human ferritin H chain expressed in Example 1 was dissolved in a pH 7.4 PBS buffer so as to have a concentration of 5.0 mg/ml. Ethyl maleimide was dissolved in N,N-dimethylformamide so as to have a concentration of 20 mM. Six equivalents (based on a molar amount, the same applies hereinafter) of ethyl maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material, and a peak at 21344 at which two molecules of ethyl maleimide (molecular weight: 125) were introduced was confirmed for the product (FIG. 2).

### Example 3: Peptide mapping of ethyl maleimide-introduced product of human ferritin H chain

### (3-1) Trypsin treatment of ethyl maleimide-introduced product of human ferritin H chain

To a 1.5 mL low-adsorption micro test tube, 10 µL of a sample solution (containing the ethyl maleimide-introduced product of a human ferritin heavy chain prepared in Example 2), 30 µL of a 50 mM tris-hydrochloric acid buffer/8M urea, and 10 µL of trifluoroethanol were added, and the resulting mixture was stirred. Thereafter, 15 µL of a 48 mM dithiothreitol aqueous solution was added thereto, and the resulting mixture was heated at 65°C for one hour. Thereafter, 15 µL of a 120 mM iodoacetamide aqueous solution was added thereto, and the resulting mixture was caused to react in a dark place at room temperature for 60 minutes. After the reaction, 5 µL of a 48 mM dithiothreitol aqueous solution was added to the solution to suppress overreaction. 150 µL of a 50 mM tris-hydrochloric acid buffer was added thereto, and the resulting mixture was stirred. 2.5 µL of a 100 ng/µL trypsin aqueous solution (Proteomics Grade, Code No. T6567-5 × 20 µg (SIGMA)) was added thereto, and the resulting mixture was allowed to stand at 37°C for one hour. Furthermore, 2.5 µL of a 100 ng/µL trypsin aqueous solution was added thereto, and the resulting mixture was subjected to enzymatic digestion at 37°C for 15 hours. After the digestion, 15 µL of a 20% trifluoroacetic acid aqueous solution was added to the solution to stop the reaction, and the resulting mixture was diluted 10 times with a 0.1% trifluoroacetic acid aqueous solution and then subjected to LC-MS/MS measurement.

### (3-2) Conditions for LC-MS/MS measurement of human ferritin heavy chain

### (Analyzer)

Nano HPLC: EASY-nLC 1000 (Thermo Fisher Scientific)
Mass Spectrometer: Tribrid Mass Spectrometer Orbitrap
Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75%
(23.5 minutes to 25.5 minutes), and 75% (25.5 minutes to 35.0 minutes).

### (Mass Spectrometer Analysis Conditions)

Ionization: ESI, Positive mode
Scan type: Data Dependent Aquisition
Activation Type: Collision Induced Dissociation(CID)

Data acquisition was performed using Xcalibur 3.0 (Thermo Fisher Scientific) and Thermo Orbitrap Fusion Tune Application 2.0 (Thermo Fisher Scientific) as accompanying software.

### (3-3) Analysis condition of modified site of human ferritin H chain

Modified site analysis of an LC-MS/MS measurement result was performed using BioPharma Finder 3.0 (Thermo Fisher Scientific).

The analysis with BioPharma Finder was performed by setting S/N threshold to 20 and setting MS Noise Level to 1000. A digestive enzyme was set to Trypsin, and Specificity was set to High. Furthermore, the Mass Accuracy at the time of peptide identification was set to 5 ppm. For Dynamic Modifications, oxidation of methionine (+15.995 Da),a modified product to a cysteine residue (N-ethyl maleimide-introduced product (+125.048Da), and carbamidomethylation with iodoacetamide (+57.021Da)) were set. In addition, a filter was set such that only those with a Confidence Score of 80 or higher and with observed MS/MS were obtained.

In addition, the amino acid sequence indicated in SEQ ID NO: 1 was used as data of an amino acid sequence to be searched for a modified site.

### (3-4) Analysis result of modified site of human ferritin H chain by LC-MS/MS

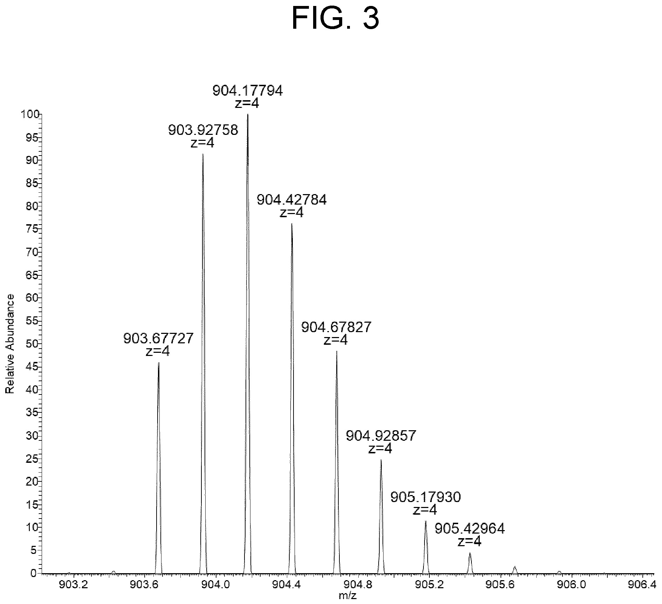

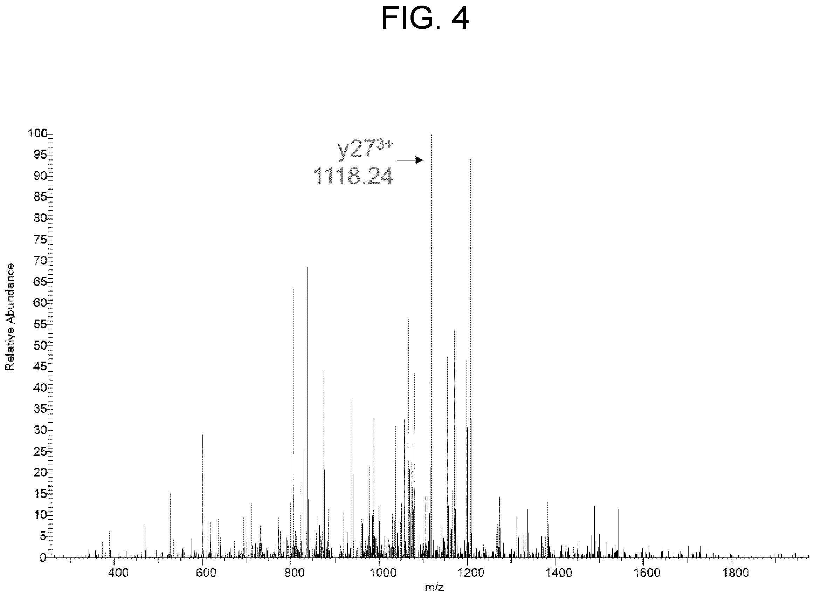

As a result of analysis using LC-MS/MS, an MS spectrum of a peptide fragment of IFLQDIKKPDCDDWESGLNAMECALHLEK (SEQ ID NO: 8), which is a peptide consisting of 29 amino acid residues containing a modified site (N-ethyl maleimide-introduced product (+125.048Da)) to two cysteine residues generated by trypsin digestion of a specific modified product of a human ferritin heavy chain by ethyl maleimide of Example 2 (measured value: m/z 903.67727, theoretical value: 903.67738, tetravalent) was observed (FIG. 3), and from a CID spectrum, a product ion of m/z 1118.24 (theoretical value: 1117.85) corresponding to trivalent y27 indicating modification of cysteine residues at positions 91 and 103 of a human ferritin H chain (at positions 90 and 102 of the amino acid sequence of SEQ ID NO: 3) was confirmed (FIG. 4).

### Example 4: Specific modification of human ferritin H chain with p-azidophenacyl bromide and ESI-TOFMS analysis

### (4-1) Specific modification of human ferritin H chain by six equivalents of p-azidophenacyl bromide and ESI-TOFMS analysis

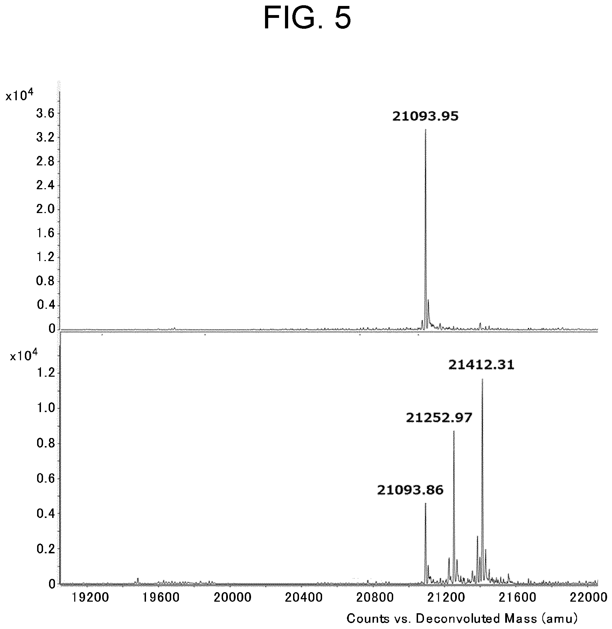

The human ferritin H chain expressed in Example 1 was dissolved in a pH 7.4 PBS buffer so as to have a concentration of 5.0 mg/ml. p-Azidophenacyl bromide was dissolved in N,N-dimethylformamide so as to have a concentration of 20 mM. Six equivalents of p-azidophenacyl bromide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. The peak of the raw material, a peak at 21252 at which one molecule of p-azidophenacyl (molecular weight: 160) was introduced, and a peak at 21412 at which two molecules of p-azidophenacyl were introduced were confirmed for the product (FIG. 5).

### (4-2) Specific modification of human ferritin H chain by 12 equivalents of p-azidophenacyl bromide and ESI-TOFMS analysis

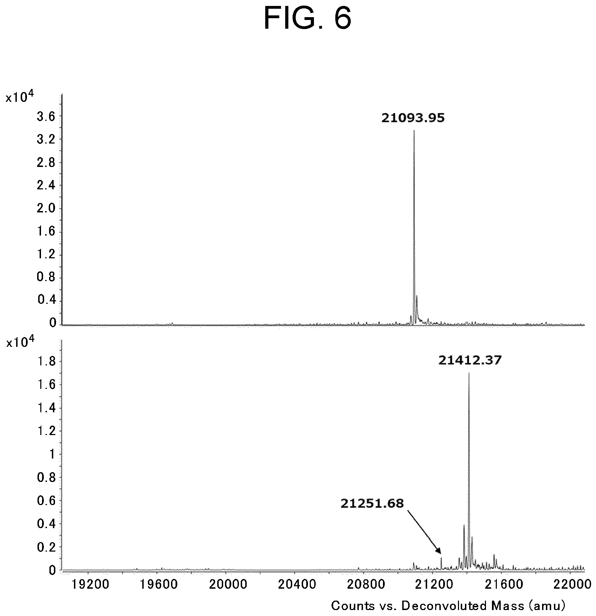

The human ferritin H chain expressed in Example 1 was dissolved in a pH 7.4 PBS buffer so as to have a concentration of 5.0 mg/ml. p-Azidophenacyl bromide was dissolved in N,N-dimethylformamide so as to have a concentration of 20 mM. 12 equivalents of p-azidophenacyl bromide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. A peak at 21252 at which one molecule of p-azidophenacyl (molecular weight: 160) was introduced, and a peak at 21412 at which two molecules of p-azidophenacyl were introduced were confirmed for the product (FIG. 6).

### Example 5: Peptide mapping of p-azidophenacyl-introduced product of human ferritin H chain

### (5-1) Trypsin treatment of p-azidophenacyl-introduced product of human ferritin H chain

A treatment similar to Example 3 (3-1) was performed except that the sample solution containing the p-azidophenacyl-introduced product of the human ferritin H chain prepared in Example 4 (4-2) was used instead of the sample solution containing the ethyl maleimide-introduced product of the human ferritin heavy chain prepared in Example 2, and the ethyl maleimide-introduced product of the human ferritin heavy chain was subjected to LC-MS/MS measurement.

### (5-2) Conditions for LC-MS/MS measurement of human ferritin H chain

An analyzer, HPLC analysis conditions, and mass spectrometer analysis conditions are the same as those in Example 3 (3-2).

### (5-3) Analysis conditions of modified site of human ferritin H chain

Modified site analysis of an LC-MS/MS measurement result was performed using BioPharma Finder 3.0 (Thermo Fisher Scientific).

The analysis with BioPharma Finder was performed by setting S/N threshold to 20 and setting MS Noise Level to 1000. A digestive enzyme was set to Trypsin, and Specificity was set to High. Furthermore, the Mass Accuracy at the time of peptide identification was set to 5 ppm. For Dynamic Modifications, oxidation of methionine (+15.995 Da), a modified product to a cysteine residue (p-azidophenacyl-introduced product (+159.043262Da), a p-aminophenacyl-introduced product (+133.052764Da), and carbamidomethylation with iodoacetamide (+57.021Da)) were set. In addition, a filter was set such that only those with a Confidence Score of 80 or higher and with observed MS/MS were obtained.

In addition, the amino acid sequence indicated in SEQ ID NO: 1 was used as data of an amino acid sequence to be searched for a modified site.

Note that since reductive alkylation with dithiothreitol and iodoacetamide is performed during digestion, the p-azidophenacyl-introduced product is partially reduced to become a p-aminophenacyl-introduced product.

### (5-4) Analysis result of modified site of human ferritin H chain by LC-MS/MS

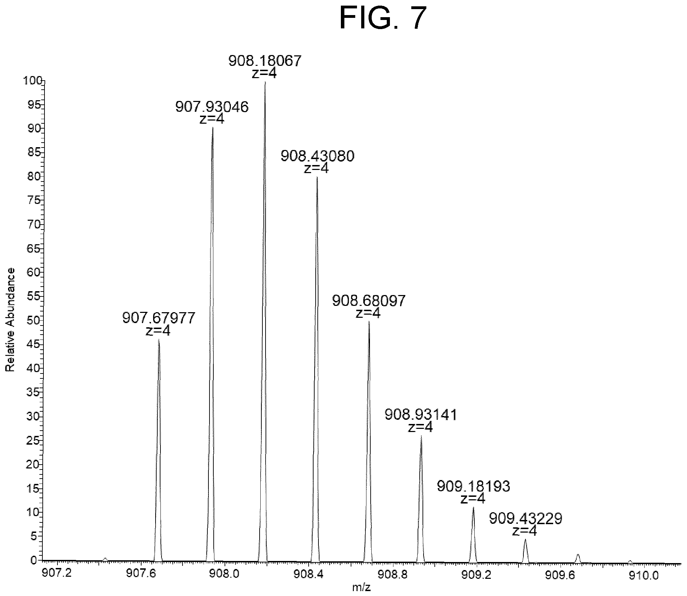

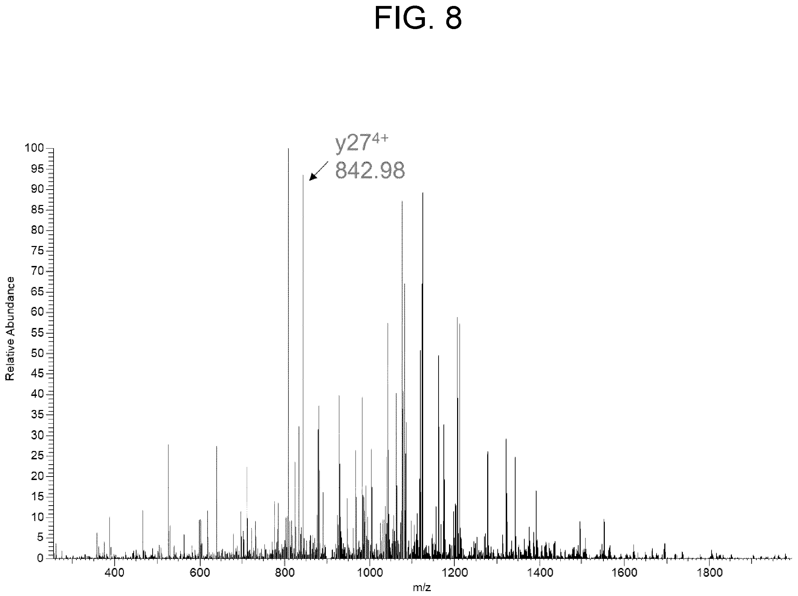

As a result of analysis using LC-MS/MS, an MS spectrum of a peptide fragment of IFLQDIKKPDCDDWESGLNAMECALHLEK (SEQ ID NO: 8), which is a peptide consisting of 29 amino acid residues containing a modified site (p-aminophenacyl-introduced product (+133.052764Da) reduced by dithiothreitol) to two cysteine residues generated by trypsin digestion of a specific modified product of a human ferritin heavy chain by p-azidophenacyl of Example 4 (4-2) (measured value: m/z 907.67977, theoretical value: 907.67992, tetravalent) was observed (FIG. 7), and from a CID spectrum, a product ion of m/z 842.98 (theoretical value: 842.64) corresponding to tetravalent y27 indicating modification of cysteine residues at positions 91 and 103 of a human ferritin H chain (at positions 90 and 102 of the amino acid sequence of SEQ ID NO: 3) was confirmed (FIG. 8) .

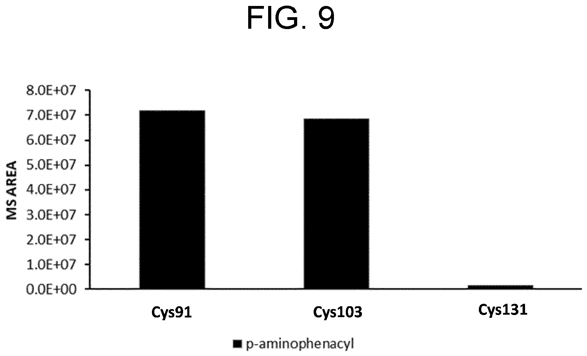

Based on the analysis result obtained in (5-4), peak area values at positions 91, 103, and 131 (at positions 90, 102, and 130 in the amino acid sequence of SEQ ID NO: 3) of the human ferritin H chain were compared (FIG. 9). It can be seen that positions 91 and 103 of the human ferritin H chain (positions 90 and 103 in the amino acid sequence of SEQ ID NO: 3) are selectively modified.

### Example 6: Specific modification of human ferritin H chain with Cy5-PEG-maleimide and ESI-TOFMS analysis

### (6-1) Specific modification of human ferritin H chain with Cy5-PEG-maleimide and ESI-TOFMS analysis

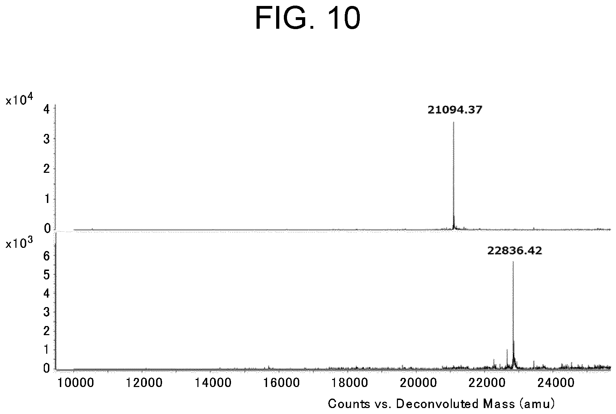

The human ferritin H chain expressed in Example 1 was dissolved in a pH 7.4 PBS buffer so as to have a concentration of 5.0 mg/ml. Cy5-PEG-maleimide (Broad Pharm, BP-23037) was dissolved in N,N-dimethylformamide so as to have a concentration of 20 mM. Six equivalents of Cy5-PEG-maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material. The peak of the raw material and a peak at 22836 at which two molecules of Cy5-PEG-maleimide (molecular weight: 871) were introduced were confirmed for the product (FIG. 10).

### Example 7: Specific modification of human ferritin H chain with DBCO-PEG4-maleimide and ESI-TOFMS analysis

### (7-1) Specific modification of human ferritin H chain with DBCO-PEG4-maleimide and ESI-TOFMS analysis

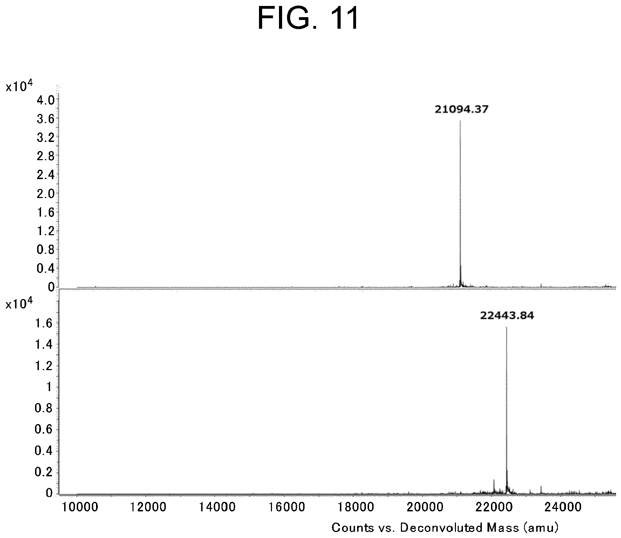

The human ferritin H chain expressed in Example 1 was dissolved in a pH 7.4 PBS buffer so as to have a concentration of 5.0 mg/ml. DBCO-PEG4-maleimide (Broad Pharm, BP-22294) was dissolved in N,N-dimethylformamide so as to have a concentration of 20 mM. Six equivalents of DBCO-PEG4-maleimide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material. The peak of the raw material and a peak at 22443 at which two molecules of DBCO-PEG4-maleimide (molecular weight: 673) were introduced were confirmed for the product (FIG. 11) .

### Example 8: Specific modification of human ferritin H chain with 1,3-dichloroacetone, oxime ligation with aminooxy-PEG5-azide, and ESI-TOFMS analysis

### (8-1) Specific modification of human ferritin H chain with 1,3-dichloroacetone and ESI-TOFMS analysis

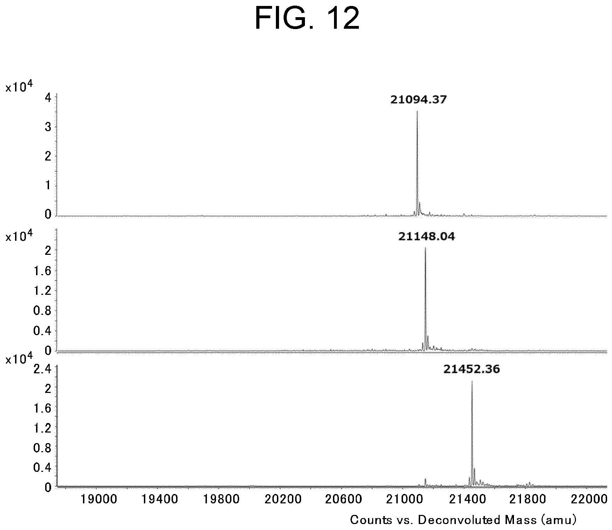

The human ferritin H chain expressed in Example 1 was dissolved in a pH 7.4 PBS buffer so as to have a concentration of 5.0 mg/ml. 1,3-Dichloroacetone was dissolved in N,N-dimethylformamide so as to have a concentration of 20 mM. Six equivalents of 1,3-dichloroacetone was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human ferritin H chain as a raw material, and a peak at 21148 at which one molecule of acetone (molecular weight: 54) was introduced for the product was confirmed (FIG. 12).

### (8-2) Specific modification of oxime ligation with aminooxy-PEG5-azide and ESI-TOFMS analysis

The human ferritin H chain acetone modified product obtained in (8-1) was dissolved in a pH 4.7 acetic acid buffer so as to have a concentration of 2.0 mg/ml. Aminooxy-PEG5-azide (Broad Pharm, BP-23194) was dissolved in N,N-dimethylformamide so as to have a concentration of 20 mM. Ten equivalents of aminooxy-PEG5-azide was added to the aqueous solution containing the human ferritin H chain acetone modified product. The resulting mixture was stirred, and then subjected to a shaking reaction at 37°C for 16 hours. The reaction solution was replaced with a 20 mM ammonium acetate buffer, and the mass was measured by ESI-TOFMS. A peak was observed at 21148 for the human ferritin H chain acetone modified product as a raw material. A peak at 21452 at which one molecule of aminooxy-PEG5-azide (molecular weight: 304) was introduced was confirmed for the product (FIG. 12).

### Example 9: Peptide mapping of 1,3-dichloroacetone-introduced product of human ferritin H chain

### (9-1) Trypsin treatment of 1,3-dichloroacetone-introduced product of human ferritin H chain

To a 1.5 mL low-adsorption micro test tube, 5 µL of a sample solution (containing the 1,3-dichloroacetone-introduced product of the human ferritin H chain prepared in Example 8), 35 µL of a 50 mM tris-hydrochloric acid buffer/8M urea, and 10 µL of trifluoroethanol were added, and the resulting mixture was stirred. Thereafter, 15 µL of a 48 mM dithiothreitol aqueous solution was added thereto, and the resulting mixture was heated at 65°C for one hour. Thereafter, 15 µL of a 120 mM iodoacetamide aqueous solution was added thereto, and the resulting mixture was caused to react in a dark place at room temperature for one hour. After the reaction, 5 µL of a 48 mM dithiothreitol aqueous solution was added to the solution to suppress overreaction. 150 µL of a 50 mM tris-hydrochloric acid buffer was added thereto, and the resulting mixture was stirred. 2.5 µL of a 100 ng/µL trypsin aqueous solution was added thereto, and the resulting mixture was allowed to stand at 37°C for one hour. Furthermore, 2.5 µL of a 100 ng/µL trypsin aqueous solution was added thereto, and the resulting mixture was subjected to enzymatic digestion at 37°C for 15 hours. After the digestion, 15 µL of a 20% trifluoroacetic acid aqueous solution was added thereto to stop the reaction, and the resulting mixture was diluted 10 times with a 0.1% formic acid aqueous solution and then subjected to LC-MS/MS measurement.

### (9-2) Conditions for LC-MS/MS measurement of human ferritin H chain

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Aquisition
Activation Type:Collision Induced Dissociation(CID)
MS 1:Orbitrap
MS2:Ion Trap

Data acquisition was performed using Xcalibur 3.0 (Thermo Fisher Scientific) and Thermo Orbitrap Fusion Tune Application 2.0 (Thermo Fisher Scientific) as accompanying software.

### (9-3) Analysis condition of modified site of human ferritin H chain

Modified site analysis of an LC-MS/MS measurement result was performed using Xcalibur 3.0 (Thermo Fisher Scientific).

In Xcalibur, an extraction chromatogram was created within a range of ± 0.005 with respect to a peptide theoretical value, and automatic peak area calculation was performed by software. Note that, when the peptide theoretical value was calculated, possibility of oxidation of a methionine residue (+15.995Da), 1,3-dichloroacetone modification to a cysteine residue (+54.011Da), and carbamide methylation by iodoacetamide (+57.021Da) was considered, and the amino acid sequence of the human ferritin H chain indicated in SEQ ID NO: 3 was used as data of an amino acid sequence.

### (9-4) Analysis result of modified site of human ferritin H chain by LC-MS/MS

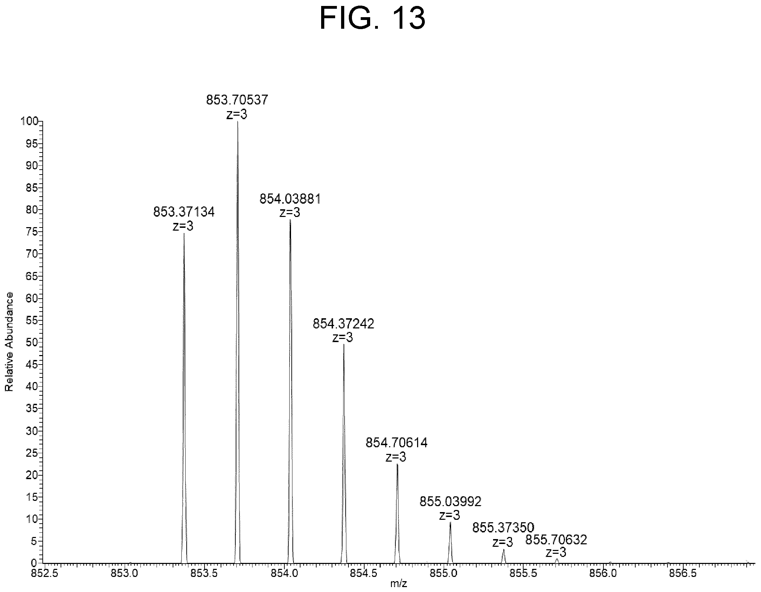

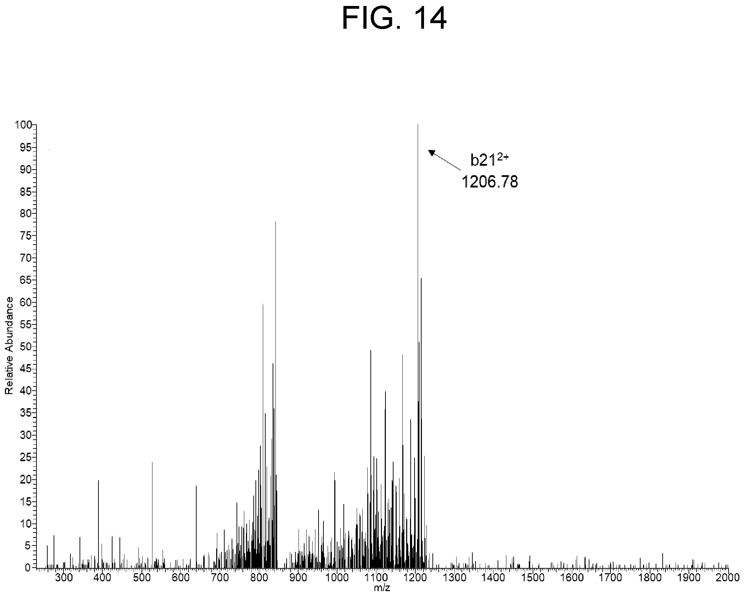

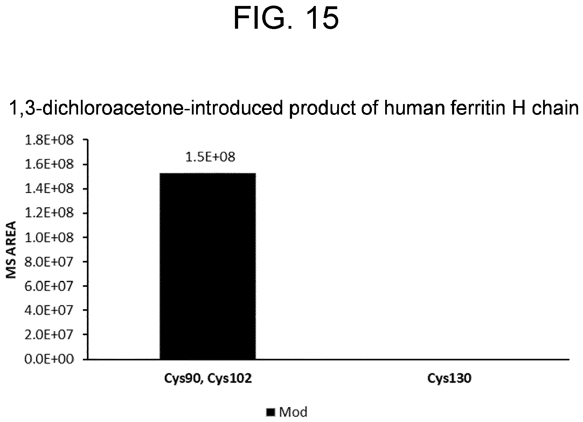

As a result of analysis using LC-MS/MS, an MS spectrum of a peptide fragment of KPDCDDWESGLNAMECALHLEK (SEQ ID NO: 9), which is a peptide consisting of 22 amino acid residues containing one modification (1,3-dichloroacetone modification (+54.011Da)) to a cysteine residue generated by trypsin digestion of a 1,3-dichloroacetone-introduced product of a human ferritin H chain in Example 8 (measured value: m/z 853.37134, theoretical value: 853.37194, trivalent) was observed (FIG. 13), and from a CID spectrum, a product ion of m/z 1206.78 (theoretical value: 1206.50) corresponding to divalent b21 indicating 1,3-dichloroacetone modification of cysteine residues at amino acid positions 90 and 102 was confirmed (FIG. 14). In addition, by peak area comparison, it was indicated that modifications to cysteine residues at amino acid positions 90 and 102 occurred with high selectivity (FIG. 15).

### Example 10: Construction of amide group-modified ferritin

### (10-1) Specific modification of human ferritin H chain with α-iodoacetamide and ESI-TOFMS analysis

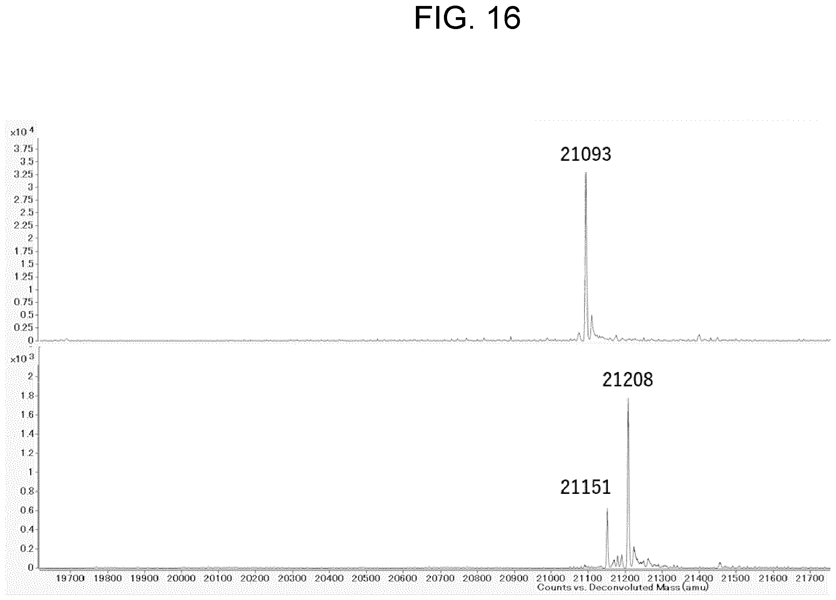

The human ferritin H chain expressed in Example 1 was dissolved in a pH 8.5 HEPES buffer so as to have a concentration of 5.0 mg/ml. α-iodoacetamide was dissolved in N,N-dimethylformamide so as to have a concentration of 50 mM. 20 equivalents of α-iodoacetamide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. The peak of the raw material, a peak at 21151 at which one molecule of p-azidophenacyl (molecular weight: 160) was introduced, and a peak at 21208 at which two molecules of p-azidophenacyl were introduced were confirmed for the product (FIG. 16).

### Example 11: Peptide mapping of α-iodoacetamide-introduced product of human ferritin H chain

### (11-1) Trypsin treatment of α-iodoacetamide-introduced product of human ferritin H chain

To a 1.5 mL low-adsorption micro test tube, 5 µL of a sample solution (containing the α-iodoacetamide-introduced product of the human ferritin H chain prepared in Example 10), 35 µL of a 50 mM tris-hydrochloric acid buffer/8M urea, and 10 µL of trifluoroethanol were added, and the resulting mixture was stirred. Thereafter, 15 µL of a 48 mM dithiothreitol aqueous solution was added thereto, and the resulting mixture was heated at 65°C for one hour. Thereafter, 15 µL of a 120 mM iodoacetic acid aqueous solution was added thereto, and the resulting mixture was caused to react in a dark place at room temperature for one hour. After the reaction, 5 µL of a 48 mM dithiothreitol aqueous solution was added to the solution to suppress overreaction. 150 µL of a 50 mM tris-hydrochloric acid buffer was added thereto, and the resulting mixture was stirred. 2.5 µL of a 100 ng/µL trypsin aqueous solution was added thereto, and the resulting mixture was allowed to stand at 37°C for one hour. Furthermore, 2.5 µL of a 100 ng/µL trypsin aqueous solution was added thereto, and the resulting mixture was subjected to enzymatic digestion at 37°C for 15 hours. After the digestion, 15 µL of a 20% trifluoroacetic acid aqueous solution was added thereto to stop the reaction, and the resulting mixture was diluted 10 times with a 0.1% formic acid aqueous solution and then subjected to LC-MS/MS measurement.

### (11-2) Conditions for LC-MS/MS measurement of human ferritin H chain

Conditions for LC-MS/MS measurement of a human ferritin H chain were similar to those in Example 9.

### (11-3) Analysis condition of modified site of human ferritin H chain

Modified site analysis of an LC-MS/MS measurement result was performed using Xcalibur 3.0 (Thermo Fisher Scientific).

In Xcalibur, an extraction chromatogram was created within a range of ± 0.005 with respect to a peptide theoretical value, and automatic peak area calculation was performed by software. Note that, when the peptide theoretical value was calculated, possibility of oxidation of a methionine residue (+15.995Da), α-iodoacetamide modification to a cysteine residue (+57.021Da), and carbamide methylation by iodoacetic acid (+58.005Da) was considered, and the amino acid sequence of the human ferritin H chain indicated in SEQ ID NO: 3 was used as data of an amino acid sequence.

### (11-4) Analysis result of modified site of human ferritin H chain by LC-MS/MS

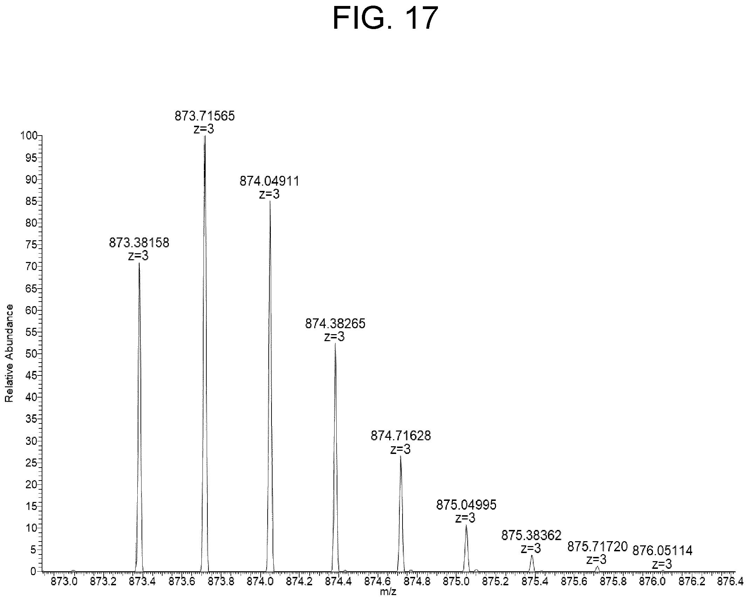

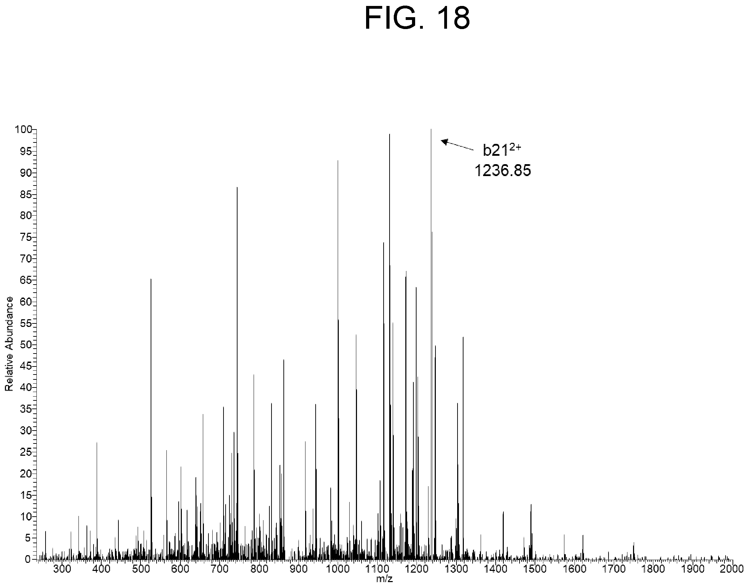

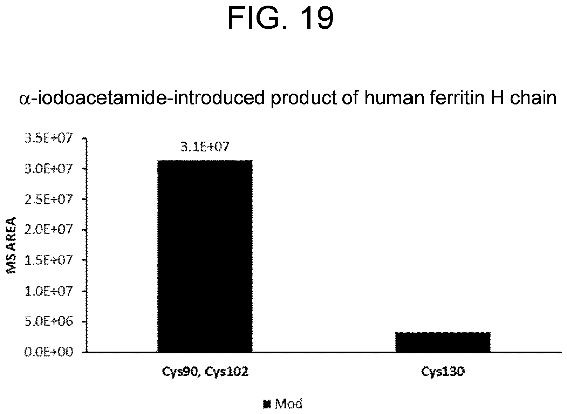

As a result of analysis using LC-MS/MS, an MS spectrum of a peptide fragment of KPDCDDWESGLNAMECALHLEK (SEQ ID NO: 9), which is a peptide consisting of 22 amino acid residues containing two modifications (α-iodoacetamide modification (+57.021Da)) to a cysteine residue generated by trypsin digestion of an α-iodoacetamide-introduced product of a human ferritin H chain in Example 10 (measured value: m/z 873.38158, theoretical value: 873.38273, trivalent) was observed (FIG. 17), and from a CID spectrum, a product ion of m/z 1236.85 (theoretical value: 1236.52) corresponding to divalent b21 indicating α-iodoacetamide modification of cysteine residues at amino acid positions 90 and 102 was confirmed (FIG. 18). In addition, by peak area comparison, it was indicated that modifications to cysteine residues at amino acid positions 90 and 102 occurred with high selectivity (FIG. 19).

### Example 12: Physical property analysis of ethyl maleimide-modified ferritin

FTH (to ethyl maleimide-modified FTH, 24-mer chemical amount: 512256) in which ethyl maleimide was introduced into cysteines at positions 91 and 103 constructed in Example 2 was suspended in D-PBS (-) (pH 7.4, FUJIFILM Wako Pure Chemical Corporation) so as to have a final concentration of 1 mg/ml. The resulting suspension was left to stand at 25°C for 30 minutes. The solution dispersibility and size of an amidated FTH contained in the solution were measured by a dynamic light scattering (DLS) method using Zetasizer Nano ZS (Malvern Panalytical Ltd.). The measurement was performed on 50 µl of the solution at 25°C.

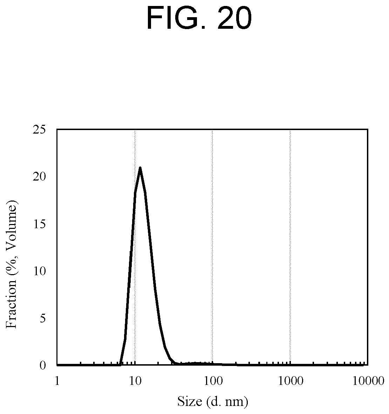

As a result, good dispersion having a peak at 11.7 nm was observed (FIG. 20), and it was found that the cage-like structure of ferritin was maintained even after modification.

The size of the ethyl maleimide-modified FTH was also evaluated by size exclusion column chromatography. 10 µl of the solution in which the ethyl maleimide-modified ferritin was suspended in D-PBS (-) so as to have a final concentration of 1 mg/ml was loaded on Superdex 200 increase 16/30 (Cytiva) and eluted at a flow rate of 0.8 ml/min in PBS (Takara Bio Inc.), and a protein was detected by light absorption at 280 nm.

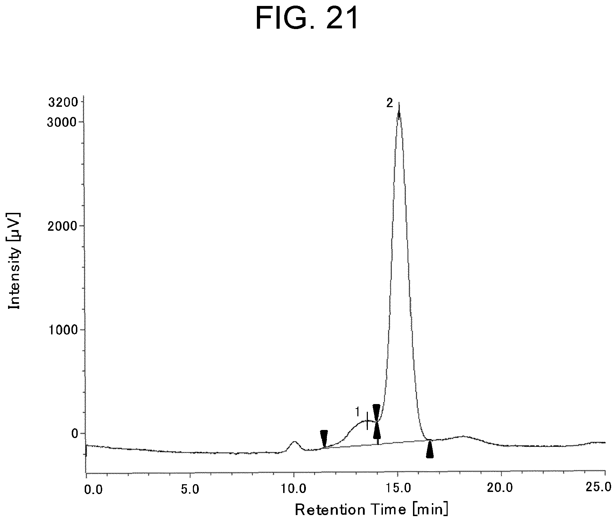

As a result, in the ethyl maleimide-modified FTH, elution in the same elution time (15 min) as in a natural FTH was confirmed, and it was found that there was no aggregation and the size was not changed (FIG. 21).

Furthermore, a surface charge of the ethyl maleimide-modified ferritin was evaluated. The ethyl maleimide-modified FTH was suspended in D-PBS (-) so as to have a final concentration of 0.1 g/l, and a surface charge at 25°C in the buffer was measured with Zetasizer Nano ZS (Malvern Panalytical Ltd.). 750 µl of a sample was put in a DTS 1070 cell, and the measurement was performed at Material setting (RI: 1.450, Absorption: 0.001), Dispersant setting (Temperature: 25°C, Viscosity: 0.8872 cP, RI: 1.330, Dielectric constant: 78.5), and Smoluchowski model (Fκa value: 1.50).

As a result, it was found that the surface charge of the ethyl maleimide-modified FTH was -17 mV, which was equivalent to a surface charge (-17 mV) of FTH in which an ethyl maleimide group was not introduced in D-PBS (-), and the surface of the FTH was not significantly changed by the modification.

### Example 13: Molecular encapsulation in ethyl maleimide-modified ferritin (1)

A low molecular anticancer agent was encapsulated in the ethyl maleimide-modified FTH constructed in Example 2. Doxorubicin hydrochloride (DOX, CAS No. 25316-40-9) was mixed in 1 ml of a 50 mM tris-hydrochloride buffer (pH 9) containing the ethyl maleimide-modified FTH at a final concentration of 1 mg/ml such that a final concentration was 0.3 mg/l, and the resulting mixture was left to stand at 60°C for 60 minutes. After the mixture was left to stand, the mixture was centrifuged (15,000 rpm, one minute). Thereafter, the supernatant was supplied to a desalting column PD-10 (packed with Sephadex G-25, Cytiva) equilibrated with a 10 mM tris-hydrochloride buffer (pH 8), and a drug and a protein which had not been encapsulated were separated. 1.0 ml out of the total amount (3.0 ml) of the solution was loaded on Superdex 200 increase 16/30 (Cytiva) and eluted in D-PBS (-) (pH 7.4, FUJIFILM Wako Pure Chemical Corporation) at a flow rate of 0.8 ml/min. A protein was purified using light absorption at 280 nm as an index to obtain doxorubicin-encapsulating ethyl maleimide-modified FTH (DOX-ethyl maleimide-modified FTH).

In order to examine stability of the obtained DOX-ethyl maleimide-modified FTH, 1 ml of D-PBS (-) containing an ethyl maleimide-modified ferritin at 0.2 mg/ml was left to stand at room temperature for 24 hours, then loaded on Superdex 200 increase 16/30 (Cytiva), and subjected to simultaneous measurement of light absorption at 280 nm and light absorption at 480 nm while being separated and purified by size at a flow rate of 0.8 ml/min in D-PBS (-) (pH 7.4, FUJIFILM Wako Pure Chemical Corporation).

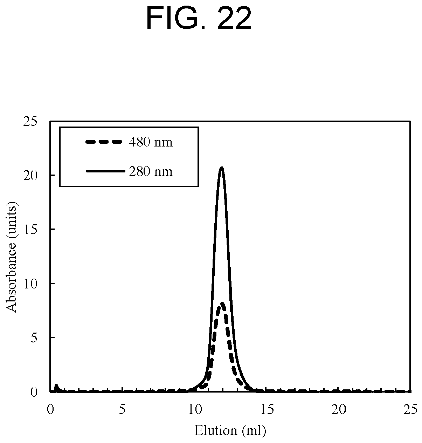

As a result, a light absorption peak at 480 nm, which is presumed to be derived from DOX, was observed at the same elution position as that of the FTH containing no DOX, and it was found that DOX and the ethyl maleimide-modified FTH were strongly complexed (FIG. 22).

Furthermore, a surface charge of the DOX-ethyl maleimide-modified FTH was evaluated. The DOX-ethylmaleimide-modified FTH was suspended in a 50 mM tris-hydrochloride buffer (pH 8.0) so as to have a final concentration of 0.1 g/l as an FTH amount, and a surface charge at 25°C in the buffer was measured with Zetasizer Nano ZS (Malvern Panalytical Ltd.). 800 µl of a sample was put in a DTS 1070 cell, and the measurement was performed at Material setting (RI: 1.450, Absorption: 0.001), Dispersant setting (Temperature: 25°C, Viscosity: 0.8872 cP, RI: 1.330, Dielectric constant: 78.5), and Smoluchowski model (Fκa value: 1.50).

As a result, it was found that the surface charge of the DOX-ethyl maleimide-modified FTH was -10.9 ± 0.5 mV (average ± standard deviation), which was equivalent to a surface charge (-11 mV) of FTH which was not ethylmaleimidized under the same conditions, and the surface of the FTH was not significantly changed by the DOX encapsulation.

### Example 14: Molecular encapsulation in ethyl maleimide-modified ferritin (2)

A fluorescently modified peptide [5(6)-FAM-CGGPKKKRKVG (SEQ ID NO: 10), FAM-SV40, chemical formula amount: 1516] was encapsulated in the ethyl maleimide-modified ferritin constructed in Example 2. 0.1 ml of 16 mM hydrochloric acid (pH 1.6) containing amidated FTH having a final concentration of 3 mg/ml and FAM-SV40 having a final concentration of 0.1 mM was left to stand at room temperature for 15 minutes. Thereafter, 50 µl of a 1 M tris-hydrochloride buffer (pH 9.0) was added thereto for neutralization, and the resulting mixture was left to stand at room temperature for three hours. After the mixture was left to stand, the volume was increased to 1 ml with water, and the ethyl maleimide-modified ferritin and peptides that had not been encapsulated were separated and purified by size at a flow rate of 0.8 ml/min using Superdex 200 increase 16/30 (Cytiva) equilibrated with D-PBS (-) (FUJIFILM Wako Pure Chemical Corporation).

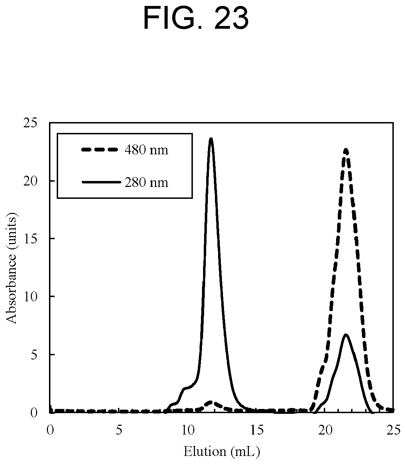

As a result, in the ferritin that had been subjected to the encapsulation operation of FAM-SV40, light absorption at 480 nm, which is not observed only for a protein, was confirmed at the same elution position as that of the ferritin 24-mer, and it was found that FAM-SV40 was encapsulated in the ethyl maleimide-modified ferritin (FIG. 23) .

### Example 15: Surface modification of molecule-encapsulating ferritin (1)

### (15-1) Chemical modification to surface of ferritin encapsulating molecule in inner cavity of ferritin

First, an FTH encapsulating a fluorescently modified peptide [(5(6)-FAM-CGGPKKKRKVG (SEQ ID NO: 10), FAM-SV40, chemical formula amount: 1516] or a double-stranded DNA [double strand of 5'-AACAGGTAGGTTCAGTGT-3' (SEQ ID NO: 11) and 5'-ACACTGAACCTACCTGTT-3' (SEQ ID NO: 12), dsDNA, chemical formula amount: 10996] was constructed.

In order to construct a ferritin encapsulating FAM-SV40 (FAM-SV40-encapsulating FTH), 1 ml of a 50 mM glycine hydrochloride buffer (pH 2.3) containing FTH (molecular weight of 24-mer: 506260) having a final concentration of 3 mg/ml and a peptide having a final concentration of 0.1 mM was left to stand at room temperature for 15 minutes. Thereafter, 10 µl of a 1 M tris-hydrochloride buffer (pH 9.0) was added thereto for neutralization, and the resulting mixture was left to stand at room temperature for three hours. Ten samples having similar reaction compositions were collected together, and the FAM-SV40-encapsulating FTH was separated and purified by size at a flow rate of 0.8 ml/min using Superdex 200 increase 16/30 (Cytiva) equilibrated with D-PBS (-) (FUJIFILM Wako Pure Chemical Corporation). The obtained sample was concentrated to 16 mg/ml as FTH with an ultrafiltration membrane (Amicon Ultra 0.5, 100 kDa, Merck) and subjected to a modification reaction.

In addition, in order to construct a ferritin encapsulating dsDNA (dsDNA-encapsulating FTH), 0.1 ml of a 50 mM glycine hydrochloride buffer (pH 2.3) containing FTH (molecular weight of 24-mer: 506260) having a final concentration of 3 mg/ml and dsDNA having a final concentration of 0.1 mM was left to stand at room temperature for 15 minutes. Thereafter, 10 µl of a 1 M tris-hydrochloride buffer (pH 9.0) was added thereto for neutralization, and the resulting mixture was left to stand at room temperature for three hours. Ten samples having similar reaction compositions were collected together, and the dsDNA-encapsulating FTH was separated and purified by size at a flow rate of 0.8 ml/min using Superdex 200 increase 16/30 (Cytiva) equilibrated with D-PBS (-) (FUJIFILM Wako Pure Chemical Corporation). The obtained sample was concentrated to 25 mg/ml as FTH with an ultrafiltration membrane (Amicon Ultra 0.5, 100 kDa, Merck) and subjected to a modification reaction.

### (15-2) Modification of fluorescently modified peptide-encapsulating ferritin with ethyl maleimide and ESI-TOFMS analysis

Subsequently, the two types of encapsulating ferritins obtained above were modified with ethyl maleimide.

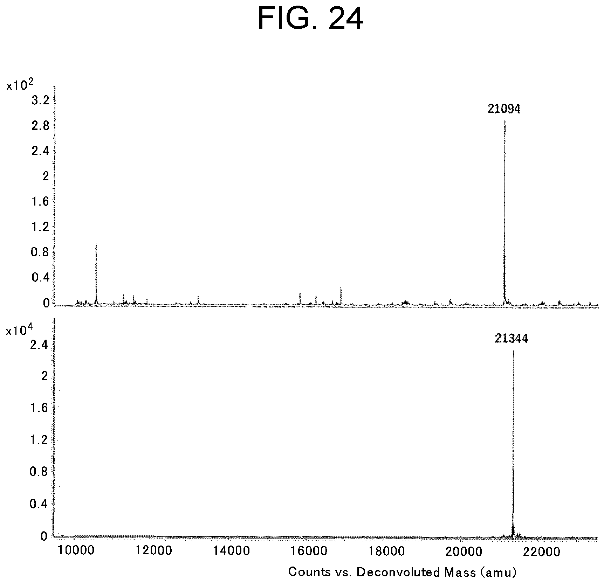

The FAM-SV40-encapsulating FTH was dissolved in a pH 7.4 PBS buffer so as to have a concentration of 5.0 mg/ml. Ethyl maleimide was dissolved in N,N-dimethylformamide so as to have a concentration of 20 mM. Six equivalents of ethyl maleimide was added to the aqueous solution containing the FAM-SV40-encapsulating FTH. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human-derived ferritin H chain as a raw material, and a peak at 21344 at which two molecules of ethyl maleimide (molecular weight: 125) were introduced was confirmed for the product (FIG. 24).

It was confirmed by size exclusion column chromatography that the FAM-SV40-encapsulating FTH maintained the FAM-SV40 encapsulation even after modification. 10 µl of a solution in which FTH was suspended in D-PBS (-) so as to have a final concentration of 2 mg/ml was loaded on Superdex 200 increase 16/30 (Cytiva) and eluted at a flow rate of 0.8 ml/min in PBS (Takara Bio Inc.). A protein was detected at 280 nm, and FAM was detected at 480 nm.

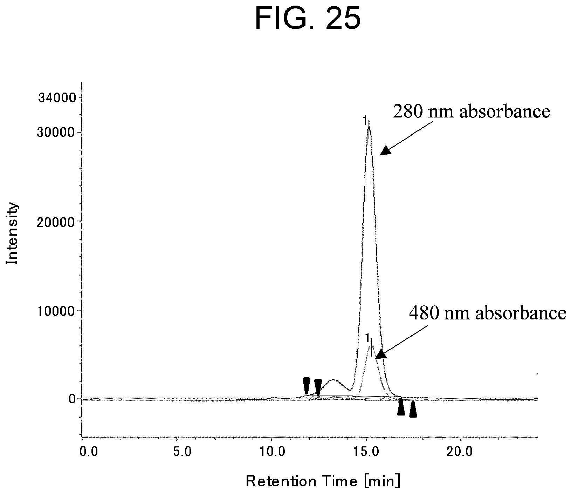

As a result, light absorption at 480 nm derived from FAM was confirmed at the same elution position as that of the ferritin 24-mer, and it was found that FAM-SV40 was maintained in ferritin even after the modification reaction (FIG. 25).

### (15-3) Modification of double-stranded DNA-encapsulating ferritin with ethyl maleimide and ESI-TOFMS analysis

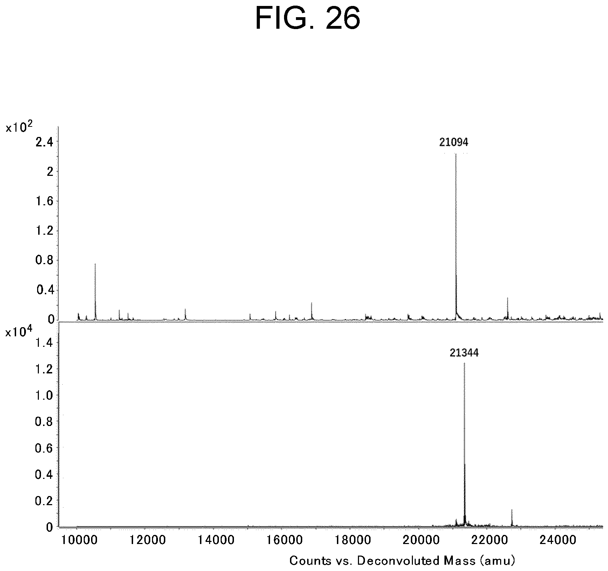

The dsDNA-encapsulating FTH was dissolved in a pH 7.4 PBS buffer so as to have a concentration of 5.0 mg/ml. Ethyl maleimide was dissolved in N,N-dimethylformamide so as to have a concentration of 20 mM. Six equivalents of ethyl maleimide was added to an aqueous solution containing the dsDNA-encapsulating FTH. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human-derived ferritin H chain as a raw material, and a peak at 21344 at which two molecules of ethyl maleimide (molecular weight: 125) were introduced was confirmed for the product (FIG. 26).

It was confirmed by size exclusion column chromatography that the dsDNA-encapsulating FTH maintained the dsDNA encapsulation even after modification. In order to confirm what was encapsulated in FTH after modification, the higher-order structure of the amidated FTH was destructed with a 100 mM glycine hydrochloride buffer (pH 2.3), and what was encapsulated in FTH was released. Thereafter, neutralization was performed with a 200 mM tris-hydrochloride buffer (pH 9.0). 10 µl of a solution in which the sample as FTH was suspended in D-PBS (-) so as to have a final concentration of 2 mg/ml was loaded on Superdex 200 increase 16/30 (Cytiva) and eluted at a flow rate of 0.8 ml/min in PBS (Takara Bio Inc.). A protein and dsDNA were detected at 280 nm.

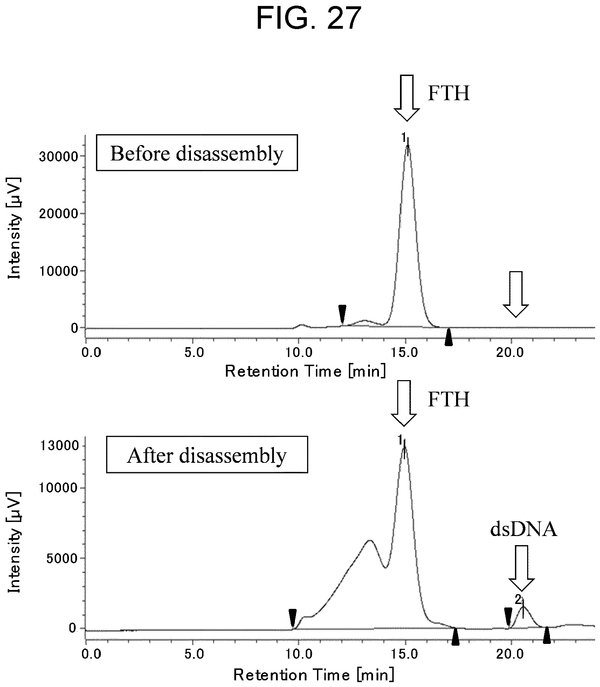

As a result, no peak was observed at an elution position (20.5 minutes) of dsDNA before the structure was destructed by the glycine hydrochloride buffer, but a peak could be observed after the structure was destructed (FIG. 27). This suggests that dsDNA inside FTH was released by the acid, and it was found that dsDNA was maintained in ferritin even after the modification reaction.

### Example 16: Surface modification of molecule-encapsulating ferritin (2)

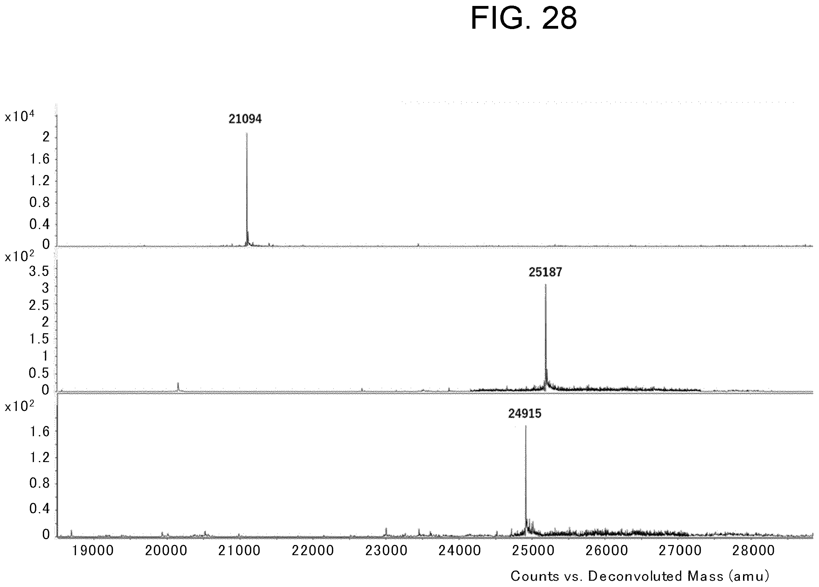

A surface of the FAM-SV40-encapsulating FTH constructed in Example 15 was modified with a peptide having a PEG6-maleimide group at a C-terminal (YGRKKRRQRRR-PEG6-maleimide, TAT-Mal, molecular weight: 2046, SEQ ID NO: 13) or (RRRRRRRRR-PEG6-maleimide, R9-Mal, molecular weight: 1910, SEQ ID NO: 14). The FAM-SV40-encapsulating FTH was dissolved in a PBS buffer (pH 7.4) so as to have a concentration of 5.0 mg/ml. TAT or R9 was dissolved in N,N-dimethylformamide so as to have a concentration of 100 mM. Ten equivalents of TAT-Mal or R9-Mal was added to the FAM-SV40-encapsulating FTH solution. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21094 for the human-derived ferritin H chain as a raw material. A peak at 25187 at which two molecules of TAT-Mal were introduced was confirmed, and a peak at 24915 at which two molecules of R9-Mal were introduced was confirmed for the product (FIG. 28).

### Example 17: Purification of azide group-modified ferritin

The azide group-modified ferritin (azidated FTH) obtained in Example 4 (4-2) was diluted with water so as to have a concentration of 1.0 mg/ml. 1.0 ml out of the solution was loaded on Superdex 200 increase 16/30 (Cytiva) and eluted in D-PBS (-) (pH 7.4, FUJIFILM Wako Pure Chemical Corporation) at a flow rate of 0.8 ml/min. A protein was purified using light absorption at 280 nm as an index to obtain azidated FTH.

### Example 18: Physical property analysis of azidated FTH

FTH which was purified in Example 17 and in which both the cysteines at positions 91 and 103 were azidated (azidated FTH, molecular weight of 24-mer: 513888)) was suspended in a 50 mM tris-hydrochloride buffer (pH 8.0) so as to have a final concentration of 0.5 mg/ml. The resulting suspension was left to stand at 25°C for 30 minutes. The solution dispersibility and size of the azidated FTH contained in the solution were measured by a dynamic light scattering (DLS) method using Zetasizer Nano ZS (Malvern Panalytical Ltd.). The measurement was performed on 50 µl of the solution at 25°C.

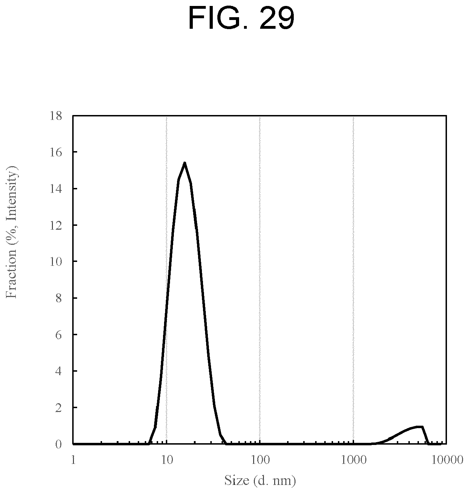

As a result, good dispersion having a peak at 16 nm (PDI0.217) was observed (FIG. 29), and it was found that the cage-like structure of ferritin was maintained even after modification.

It was also confirmed by size exclusion column chromatography that the size of the azidated FTH was maintained. 10 µl of a solution in which the azidated FTH was suspended in D-PBS (-) so as to have a final concentration of 1 mg/ml was loaded on Superdex 200 increase 16/30 (Cytiva) and eluted at a flow rate of 0.8 ml/min in PBS (Takara Bio Inc.), and detection was performed by light absorption at 280 nm and 480 nm.

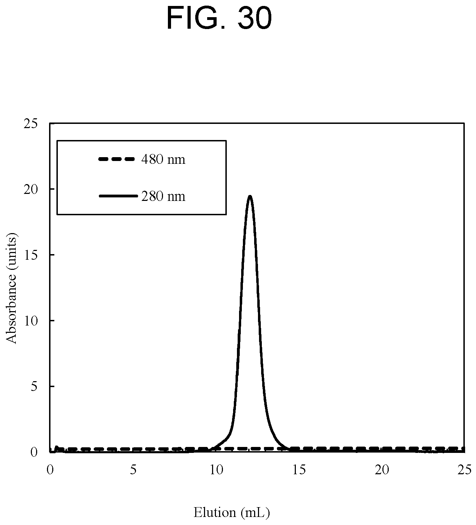

As a result, in the azidated FTH, elution in the same elution time (15 min) as that of a natural FTH was confirmed, and it was found that the size was not changed due to aggregation or the like (FIG. 30). In addition, since no component such as a dye was contained, light absorption at 480 nm was not confirmed.

Furthermore, a surface charge of the azidated FTH was evaluated. The azidated FTH was suspended in a 0 mM tris-hydrochloride buffer (pH 8.0) so as to have a final concentration of 0.1 g/l as an FTH amount, and a surface charge at 25°C in the buffer was measured with Zetasizer Nano ZS (Malvern Panalytical Ltd.). 750 µl of a sample was put in a DTS 1070 cell, and the measurement was performed at Material setting (RI: 1.450, Absorption: 0.001), Dispersant setting (Temperature: 25°C, Viscosity: 0.8872 cP, RI: 1.330, Dielectric constant: 78.5), and Smoluchowski model (Fκa value: 1.50).

As a result, it was found that the surface charge of the azidated FTH was -9.9 ± 0.9 mV, which was equivalent to a surface charge (-11 mV) of FTH not azidated, and the surface of the FTH was not significantly changed by the azidation.

### Example 19: Low molecule encapsulation in azide group-modified ferritin

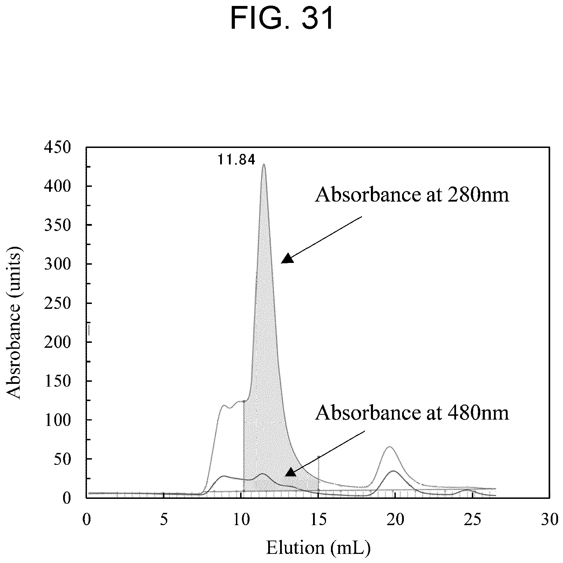

A fluorescent dye was encapsulated in the azidated FTH purified in Example 17. Uranin (CAS No. 518-47-8) was mixed in 1 ml of a 50 mM acetate buffer (pH 5) containing the azidated FTH at a final concentration of 3 mg/ml so as to have a final concentration of 100 mM, and the resulting mixture was left to stand at 40°C for 60 minutes. After the mixture was left to stand, the mixture was centrifuged (15,000 rpm, one minute). Thereafter, the supernatant was supplied to a desalting column PD-10 (packed with Sephadex G-25, Cytiva) equilibrated with D-PBS(-) (pH 7.4, FUJIFILM Wako Pure Chemical Corporation), and a drug and a protein which had not been encapsulated were separated. The total amount (3.0 ml) of the solution was concentrated by ultrafiltration using a Vivaspin 20 (MW: 100 kDa). Thereafter, 1.0 ml out of the solution was loaded on Superdex 200 increase 16/30 (Cytiva) and eluted in D-PBS (-) (pH 7.4, FUJIFILM Wako Pure Chemical Corporation) at a flow rate of 0.8 ml/min. A protein was purified using light absorption at 280 nm as an index. At the time of purification, light absorption at 480 nm, which was not observed only for a protein, was confirmed at the same elution position as that of the ferritin 24-mer, and it was found that a uranine-encapsulating azidated FTH was obtained (FIG. 31).

### Example 20: Medium molecular surface modification of ferritin

Surfaces of a natural FTH and an azidated FTH were modified with siRNA. First, siRNA (maleimide-PPIB or DBCO-PPIB) was synthesized in which A(M)C(M)AGC(M)AAAU(M)U(M)C(M)C(M)AU(M)C(M)GU(M)GU(M) (SEQ ID NO: 15, A(M), C(M), and U(M) are 2'-OMe-converted RNAs) having 6-FAM modified at a 5'-terminal and having a maleimide group or a dibenzocyclooctyne group modified at a 3'-terminal via an amino C6 linker was used as a sense strand, and 5'-AC(F)AC(F)GAU(F)GGAAU(F)U(F)U(F)GC(F)U(F)GU(F)UU-3' (SEQ ID NO: 16, C(F) and U(F) are 2'-fluorinated RNAs) was used as an antisense strand.

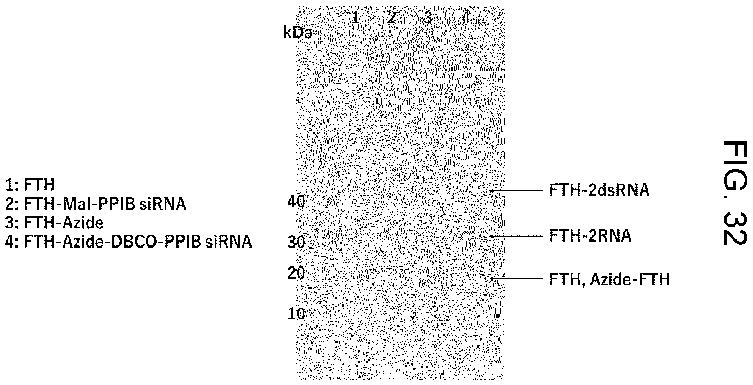

A natural FTH was dissolved in a pH 7.4 PBS buffer so as to have a concentration of 3.0 mg/ml. The above-synthesized siRNA (maleimide-PPIB) having a maleimide group at a 3'-terminal via an amino C6 linker was dissolved in N,N-dimethylformamide so as to have a concentration of 7.5 mM. 7.5 equivalents of siRNA was added to the aqueous solution containing the natural ferritin. The resulting mixture was stirred, and then subjected to a shaking reaction at room temperature for 18 hours. The reaction solution was purified by a NAP column (Cytiva). The purified product was mixed with a sample loading buffer (Bio-rad), allowed to stand at 90°C for two minutes, and then subjected to SDS-PAGE using Miniprothian TGX gel (Bio-rad). In FIG. 32, lane 1 represents the natural FTH, and lane 2 represents a product. In lane 2, one in which two sense strands were bonded to the natural FTH and one in which two dsDNAs were bonded to the natural FTH were identified.

The azidated FTH obtained in Example 4 (4-2) was dissolved in a pH 7.4 PBS buffer so as to have a concentration of 3.0 mg/ml. The above-synthesized siRNA (DBCO-PPIB) having a DBCO group at a 3'-terminal via an amino C6 linker was dissolved in N,N-dimethylformamide so as to have a concentration of 7.5 mM. 7.5 equivalents of siRNA was added to the aqueous solution containing the natural ferritin. The resulting mixture was stirred, and then subjected to a shaking reaction at room temperature for 18 hours. The reaction solution was purified by a NAP column (Cytiva). The purified product was mixed with a sample loading buffer (Bio-rad), allowed to stand at 90°C for two minutes, and then subjected to SDS-PAGE using Miniprothian TGX gel (Bio-rad). In FIG. 32, lane 3 represents the azidated FTH, and lane 4 represents a product. In lane 4, one in which two sense strands were bonded to the natural FTH and one in which two dsDNAs were bonded to the natural FTH were identified.

### Example 21: Physical property analysis of medium modified ferritin

The natural FTH and the azidated FTH with surfaces modified with siRNA constructed in Example 20 were suspended in D-PBS (-) (pH 7.4, FUJIFILM Wako Pure Chemical Corporation) so as to have a final concentration of 0.5 mg/ml. The resulting suspension was left to stand at 25°C for 30 minutes. The solution dispersibility and size of an amidated FTH contained in the solution were measured by a dynamic light scattering (DLS) method using Zetasizer Nano ZS (Malvern Panalytical Ltd.). The measurement was performed on 50 µl of the solution at 25°C.

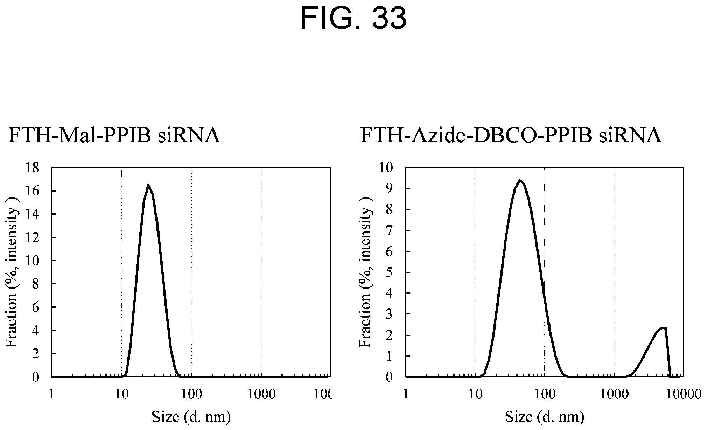

As a result, good dispersion having a peak at 16.5 nm or 50.7 nm was observed (FIG. 33), and it was suggested that the supermolecular structure of ferritin was maintained even after modification.

The size of the nucleic acid-modified FTH was also evaluated by size exclusion column chromatography. 10 µl of the solution in which the natural FTH and the azidated FTH modified with siRNA were suspended in D-PBS (-) so as to each have a final concentration of 0.5 mg/ml was loaded on Superdex 200 increase 16/30 (Cytiva) and eluted at a flow rate of 0.8 ml/min in PBS (Takara Bio Inc.), and a protein was detected by light absorption at 280 nm.

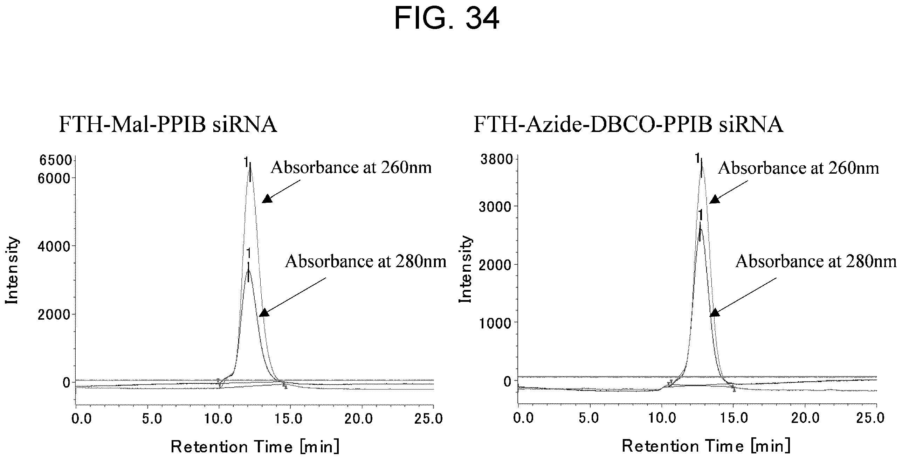

As a result, elution at a time (13 min) earlier than that of the natural FTH was confirmed, and it was found that the size was increased by the amount of the nucleic acid modified on the surface although aggregation did not occur (FIG. 34). In addition, light absorption at 260 nm larger than that of the natural FTH was observed, and it was found that absorbance at 260 nm was improved by the amount of modification with the nucleic acid.

### Example 22: Preparation of peptide-inserted human ferritin H chain

DNA encoding a human-derived ferritin H chain (FTH-BC-GBP (SEQ ID NOs: 18 and 19)) in which a gold recognition peptide (GBP1: GMHGKTQATSGTIQSG (SEQ ID NO: 17)) was inserted into and fused to a flexible linker region between the second and the third from an N-terminal among six α-helices constituting a ferritin monomer was totally synthesized. When an expression plasmid of this DNA is introduced into E. coli to express the human ferritin H chain in E. coli, methionine at an initiation codon becomes N-formylmethionine and is removed by post-translational modification. Therefore, the peptide-inserted human ferritin H chain defined by the amino acid sequence of SEQ ID NO: 20 from which a methionine residue at position 1 in the amino acid sequence of SEQ ID NO: 19 has been removed is obtained.

PCR was performed using synthetic DNA encoding FTH-BC-GBP as a template and using 5'-GAAGGAGATATACATATGACGACCGCGTCCACCTCG-3' (SEQ ID NO: 4) and 5'-CTCGAATTCGGATCCTTAGCTTTCATTATCACTGTC-3' (SEQ ID NO: 5) as primers. In addition, PCR was performed using pET20 (Merck) as a template and using 5'-TTTCATATGTATATCTCCTTCTTAAAGTTAAAC-3' (SEQ ID NO. 6) and 5'-TTTGGATCCGAATTCGAGCTCCGTCG-3' (SEQ ID NO. 7) as primers. Each of the obtained PCR products was purified with a Wizard DNA Clean-Up System (Promega Corporation), and then subjected to In-Fusion enzyme treatment with In-Fusion HD Cloning Kit (Takara Bio Inc.) at 50°C for 15 minutes, thereby constructing an expression plasmid (pET20-FTH-BC-GBP) on which a gene encoding FTH was mounted.

Subsequently, Escherichia coli BL21 (DE3) into which the constructed pET20-FTH-BC-GBP was introduced was cultured in a flask containing 100 ml of LB medium (containing 10 g/l Bacto-typtone, 5 g/l Bacto-yeast extract, 5 g/l NaCl, and 100 mg/l ampicillin) at 37°C for 24 hours. The obtained bacterial cells were ultrasonically disrupted, and then the supernatant was heated at 60°C for 20 minutes. After heating, the supernatant obtained by cooling and centrifugation was injected into a HiPerp Q HP column (Cytiva) equilibrated with a 50 mM tris-HCl buffer (pH 8.0), and subjected to a salt concentration gradient with a 50 mM tris-HCl buffer (pH 8.0) containing 0 mM to 500 mM NaCl, thereby separating and purifying a target protein due to a difference in surface charge. A solvent of a solution containing the protein was replaced with a 10 mM tris-HCl buffer (pH 8.0) by centrifugal ultrafiltration using Vivaspn 20-100K (Cytiva). The solution was injected into a HiPrep 26/60 Sephacryl S-300 HR column (Cytiva) equilibrated with a 10 mM tris-HCl buffer (pH 8.0), and FTH-BC-GBP was separated and purified. A solution containing the FTH-BC-GBP was concentrated by centrifugal ultrafiltration using Vivaspn 20-100K (Cytiva), and the concentration of a protein contained was determined by a protein assay CBB solution (Nacalai Tesque, Inc.) using bovine albumin as a standard. As a result, 1 ml of a solution containing 1 mg/ml FTH could be obtained per 100 ml of the culture solution.

### Example 23: Specific modification of peptide-inserted human ferritin H chain with p-azidophenacyl bromide and ESI-TOFMS analysis

### (23-1) Specific modification of peptide-inserted human ferritin H chain with 2.2 equivalents of p-azidophenacyl bromide and ESI-TOFMS analysis

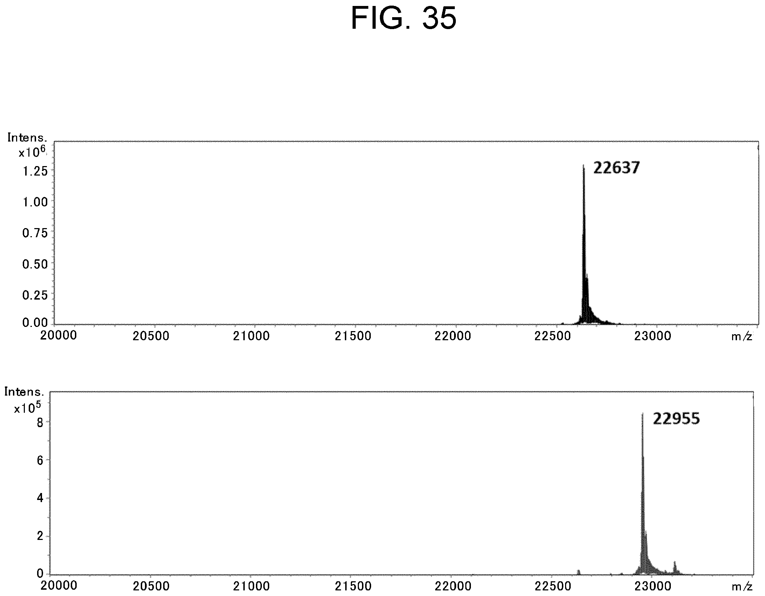

The human ferritin H chain expressed in Example 22 was dissolved in a pH 7.4 PBS buffer so as to have a concentration of 5.0 mg/ml. p-Azidophenacyl bromide was dissolved in N,N-dimethylformamide so as to have a concentration of 20 mM. 2.2 equivalents of p-azidophenacyl bromide was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 22637 for FTH-BC-GBP as a raw material. The peak of the raw material and a peak at 22955 at which two molecules of p-azidophenacyl (molecular weight: 160) were introduced were confirmed for the product (FIG. 35).

Reference Example 1: Modification of lysine residue with NHS active ester of human ferritin H chain

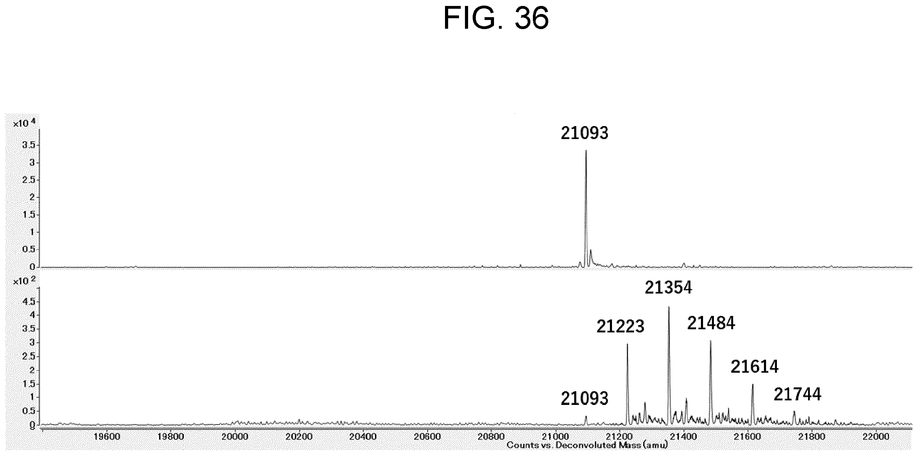

The human ferritin H chain expressed in Example 1 was dissolved in a pH 8.5 PBS buffer so as to have a concentration of 2.5 mg/ml. N-Succinimidyl 3-(acetylthio) propionate (TCI) was dissolved in N,N-dimethylformamide so as to have a concentration of 50 mM. 20 equivalents of N-succinimidyl 3-(acetylthio) propionate was added to the aqueous solution containing the human ferritin H chain. The resulting mixture was stirred, and then subjected to a shaking reaction at room temperature for one hour. The reaction solution was replaced with a 20 mM ammonium acetate buffer, and the mass was measured by ESI-TOFMS. A peak was observed at 21093 for the human ferritin H chain as a raw material. The peak of the raw material, a peak at 21223 at which one molecule of 3-(acetylthio) propionate (molecular weight: 130) was introduced, a peak at 21354 at which two molecules of 3-(acetylthio) propionate were introduced, a peak at 21484 at which three molecules of 3-(acetylthio) propionate were introduced, a peak at 21614 at which four molecules of 3-(acetylthio) propionate were introduced, and a peak at 21744 at which five molecules of 3-(acetylthio) propionate were introduced were confirmed for the product (FIG. 36).

## Claims

1. A modified ferritin comprising
a human ferritin H chain, wherein
the human ferritin H chain contains a modifying group covalently bonded specifically to a cysteine residue at position 91 and/or position 103 according to a reference position of a natural human ferritin H chain.

2. The modified ferritin according to claim 1, comprising one or two modifying groups per human ferritin H chain.

3. The modified ferritin according to claim 1 or 2, which is a 24-mer containing 24 human ferritin H chains.

4. The modified ferritin according to claim 3, comprising 24 or 48 modifying groups.

5. The modified ferritin according to any one of claims 1 to 4, wherein the human ferritin H chain is as follows:
(A) a protein comprising the amino acid sequence of SEQ ID NO: 1 or SEQ ID NO: 3;
(B) a protein comprising an amino acid sequence comprising one or several mutations of amino acid residues selected from the group consisting of replacement, deletion, insertion, and addition of amino acid residues in the amino acid sequence of SEQ ID NO: 1 or SEQ ID NO: 3 and having a 24-mer forming ability; or
(C) a protein comprising an amino acid sequence having 90% or more identity to the amino acid sequence of SEQ ID NO: 1 or SEQ ID NO: 3 and having a 24-mer forming ability.

6. The modified ferritin according to any one of claims 1 to 5, wherein the human ferritin H chain is a natural human ferritin H chain optionally having a deletion of a methionine residue at position 1.

7. The modified ferritin according to any one of claims 1 to 6, wherein the human ferritin H chain has a functional peptide inserted into a flexible linker region consisting of amino acid residues at positions 78 to 96 according to a reference position of a natural human ferritin H chain.

8. The modified ferritin according to claim 7, wherein the functional peptide is a peptide having a binding ability to a target material.

9. The modified ferritin according to any one of claims 1 to 8, wherein the modifying group contains a reactive group, and the modified ferritin is a reactive group-added ferritin.

10. The modified ferritin according to claim 9, wherein the reactive group is a bioorthogonal functional group for a protein.

11. The modified ferritin according to claim 10, wherein the bioorthogonal functional group contains one or more partial structures selected from the group consisting of a maleimide moiety, an azide moiety, a ketone moiety, an aldehyde moiety, a thiol moiety, an alkene moiety, an alkyne moiety, a halogen moiety, a tetrazine moiety, a nitrone moiety, a hydroxylamine moiety, a nitrile moiety, a hydrazine moiety, a boronic acid moiety, a cyanobenzothiazole moiety, an allyl moiety, a phosphine moiety, a disulfide moiety, a thioester moiety, an α-halocarbonyl moiety, an isonitrile moiety, a sydnone moiety, and a selenium moiety.

12. The modified ferritin according to any one of claims 1 to 8, wherein the modifying group contains a functional substance, and the modified ferritin is a functional substance-added ferritin.

13. The modified ferritin according to claim 12, wherein the functional substance contains one or more moieties selected from the group consisting of a peptide, a protein, a nucleic acid, a low molecular organic compound, a chelator, a sugar chain, a lipid, a high molecular polymer, and a metal.

14. The modified ferritin according to any one of claims 1 to 13, comprising a substance in an internal cavity thereof.

15. A method for producing a reactive group-added ferritin, comprising
reacting a human ferritin with a thiol modifying reagent to form a reactive group-added ferritin, wherein
the human ferritin and the reactive group-added ferritin each contain a human ferritin H chain, and
the human ferritin H chain contained in the reactive group-added ferritin contains a reactive group-containing modifying group covalently bonded specifically to a cysteine residue at position 91 and/or position 103 according to a reference position of a natural human ferritin H chain.

16. The method according to claim 15, wherein the thiol modifying reagent contains one or more partial structures selected from the group consisting of a maleimide moiety, a benzyl halide moiety, an α-haloamide moiety, an α-haloketone moiety, an alkene moiety, an alkyne moiety, a fluoroaryl moiety, a nitroaryl moiety, a methylsulfonyloxadiazole moiety, and a disulfide moiety.

17. A method for producing a functional substance-added ferritin, comprising
reacting a human ferritin with a functional substance to form a functional substance-added ferritin, wherein
the human ferritin and the functional substance-added ferritin each contain a human ferritin H chain, and
the human ferritin H chain contained in the functional substance-added ferritin contains a functional substance-containing modifying group covalently bonded specifically to a cysteine residue at position 91 and/or position 103 according to a reference position of a natural human ferritin H chain.

18. A method for producing a functional substance-added ferritin, comprising:
(1) reacting a human ferritin with a thiol modifying reagent to form a reactive group-added ferritin; and
(2) reacting a reactive group-added ferritin with a functional substance to form a functional substance-added ferritin, wherein
the human ferritin, the reactive group-added ferritin, and the functional substance-added ferritin each contain a human ferritin H chain,
the human ferritin H chain contained in the reactive group-added ferritin contains a reactive group-containing modifying group covalently bonded specifically to a cysteine residue at position 91 and/or position 103 according to a reference position of a natural human ferritin H chain, and
the human ferritin H chain contained in the functional substance-added ferritin contains a functional substance-containing modifying group covalently bonded specifically to a cysteine residue at position 91 and/or position 103 according to the reference position of the natural human ferritin H chain.
